# EUROPEAN PATENT APPLICATION

(11) **EP 4 582 092 A1**
(43) Date of publication of application: **09.07.2025**
(21) Application number: 23877311.3
(22) Date of filing: 11.10.2023
(51) Int. Cl.: A61K 38/00, A61K 9/08, A61K 9/16, A61K 38/08, A61K 38/10, A61K 38/16, A61K 47/18, A61K 47/20, A61K 47/34, A61K 47/38, A61P 43/00, C07K 7/64, C08L 89/00, C08L 101/00

(54) **COMPOSITION CONTAINING PEPTIDE, SURFACTANT, AND POLYMER**

(30) Priority: 12.10.2022 WO PCT/JP2022/038128; 12.10.2022 EP 22200995; 12.10.2022 TW 111138589; 28.12.2022 JP 2022211372; 19.04.2023 JP 2023068715
(71) Applicant: CHUGAI SEIYAKU KABUSHIKI KAISHA, Tokyo 115-8543 (JP)
(72) Inventor: SAKAGUCHI Mana, Yokohama City, Kanagawa 244-8602 (JP); IWATA Tomoya, Gotemba-shi, Shizuoka 412-8513 (JP); SHINKAI Hiroki, Tokyo 115-8543 (JP)
(74) Representative: Vossius & Partner Patentanwälte Rechtsanwälte mbB
(86) International application number: PCT/JP2023/036892
(87) International publication number: WO 2024/080308

(57) **Abstract**

Disclosed is a composition containing a peptide, a specific type of surfactant, and a specific type of polymer.

## Description

### [Technical Field]

The present invention relates to a composition containing a peptide, a surfactant and a polymer.

### [Background Art]

In recent years, the development of drug discovery technology that makes it possible to discover drugs toward tough targets such as protein-protein interaction inhibition, agonists, and molecular chaperones by using medium molecular compounds (e.g., having molecular weights of 500 to 2,000) has gained attention (Non Patent Document 1).

One of factors that influence a therapeutic effect of a pharmaceutical composition is solubility of an administered active ingredient in the body. In particular, peptides which are middle-molecule compounds and have recently attracted attention in development of drug discovery technology are known to significantly vary in solubility in an aqueous medium such as body fluid depending on the types of amino acid residues contained, the number of the residues, and the like.

### [Citation List]

### [Non Patent Literature]

[Non Patent Literature 1] FutureMed. Chem., 2009, 1, 1289-1310.

### [Summary of Invention]

### [Technical Problem]

It is desirable that a pharmaceutical composition containing a peptide as an active ingredient has enhanced solubility of the peptide from the viewpoint that the administered peptide exhibits drug efficacy with high efficiency in the body.

Combining a surfactant and a polymer to enhance solubility of a peptide has not been heretofore known. Combining a surfactant and a polymer to enhance solubility of a peptide without relying on the crystallization suppressing action of the polymer has not been heretofore known.

It is an object of the present invention to provide a composition containing a peptide, which has enhanced solubility of the peptide.

### [Solution to Problem]

The present inventors have found that a composition containing a peptide, a specific type of surfactant and a specific type of polymer has enhanced solubility of the peptide. Further, the present inventors have found that by such a composition, solubility of a peptide is enhanced without relying on the crystallization suppressing action of a polymer.

The present invention relates to, for example, the following.
[1] A composition comprising: a solid dispersion that is a spray-dried product of a mixture containing a peptide and one or more polymers selected from the group consisting of the following (IV) to (VIII); and one or more surfactants selected from the group consisting of the following (I) to (III):
   (I) an anionic surfactant;
   (II) an amphoteric surfactant;
   (III) a nonionic surfactant;
   (IV) hydroxypropyl methyl cellulose or a derivative thereof;
   (V) a copolymer of a (meth)acrylic acid alkyl ester and at least one monomer selected from the group consisting of a (meth)acrylic acid, a (meth)acrylic acid alkyl ester, a (meth)acrylic acid alkylaminoalkyl ester and a (meth)acrylic acid ammonioalkyl ester;
   (VI) copovidone;
   (VII) a polyvinylcaprolactam-polyvinyl acetate-polyethylene glycol graft copolymer; and
   (VIII) polyvinylpyrrolidone.
[2] A composition that is a solid dispersion, which comprises a spray-dried product of a mixture containing a peptide and one or more polymers selected from the group consisting of the following (IV) to (VIII), and is intended to be used in combination with one or more surfactants selected from the group consisting of the following (I) to (III):
   (I) an anionic surfactant;
   (II) an amphoteric surfactant;
   (III) a nonionic surfactant;
   (IV) hydroxypropyl methyl cellulose or a derivative thereof;
   (V) a copolymer of a (meth)acrylic acid alkyl ester and at least one monomer selected from the group consisting of a (meth)acrylic acid, a (meth)acrylic acid alkyl ester, a (meth)acrylic acid alkylaminoalkyl ester and a (meth)acrylic acid ammonioalkyl ester;
   (VI) copovidone;
   (VII) a polyvinylcaprolactam-polyvinyl acetate-polyethylene glycol graft copolymer; and
   (VIII) polyvinylpyrrolidone.
[3] A composition which comprises one or more surfactants selected from the group consisting of the following (I) to (III), and is intended to be used in combination with a solid dispersion that is a spray-dried product of a mixture containing a peptide and one or more polymers selected from the group consisting of the following (IV) to (VIII):
   (I) an anionic surfactant;
   (II) an amphoteric surfactant;
   (III) a nonionic surfactant;
   (IV) hydroxypropyl methyl cellulose or a derivative thereof;
   (V) a copolymer of a (meth)acrylic acid alkyl ester and at least one monomer selected from the group consisting of a (meth)acrylic acid, a (meth)acrylic acid alkyl ester, a (meth)acrylic acid alkylaminoalkyl ester and a (meth)acrylic acid ammonioalkyl ester;
   (VI) copovidone;
   (VII) a polyvinylcaprolactam-polyvinyl acetate-polyethylene glycol graft copolymer; and
   (VIII) polyvinylpyrrolidone.
[4] A composition comprising: a solid dispersion containing a peptide and one or more polymers selected from the group consisting of the following (IV) and (VII); and one or more surfactants selected from the group consisting of the following (I) to (III):
   (I) an anionic surfactant;
   (II) an amphoteric surfactant;
   (III) a nonionic surfactant;
   (IV) hydroxypropyl methyl cellulose or a derivative thereof; and
   (VII) a polyvinylcaprolactam-polyvinyl acetate-polyethylene glycol graft copolymer.
[5] A composition that is a solid dispersion, which comprises a peptide and one or more polymers selected from the group consisting of the following (IV) and (VII), and is intended to be used in combination with one or more surfactants selected from the group consisting of the following (I) to (III):
   (I) an anionic surfactant;
   (II) an amphoteric surfactant;
   (III) a nonionic surfactant;
   (IV) hydroxypropyl methyl cellulose or a derivative thereof; and
   (VII) a polyvinylcaprolactam-polyvinyl acetate-polyethylene glycol graft copolymer.
[6] A composition which comprises one or more surfactants selected from the group consisting of the following (I) to (III), and is intended to be used in combination with a solid dispersion containing a peptide and one or more polymers selected from the group consisting of the following (IV) and (VII):
   (I) an anionic surfactant;
   (II) an amphoteric surfactant;
   (III) a nonionic surfactant;
   (IV) hydroxypropyl methyl cellulose or a derivative thereof;
   (V) a copolymer of a (meth)acrylic acid alkyl ester and at least one monomer selected from the group consisting of a (meth)acrylic acid, a (meth)acrylic acid alkyl ester, a (meth)acrylic acid alkylaminoalkyl ester and a (meth)acrylic acid ammonioalkyl ester;
   (VI) copovidone;
   (VII) a polyvinylcaprolactam-polyvinyl acetate-polyethylene glycol graft copolymer; and
   (VIII) polyvinylpyrrolidone.
[7] A composition comprising: a peptide (CP02) represented by the following formula; one or more surfactants selected from the group consisting of the following (I) to (III); and one or more polymers selected from the group consisting of the following (IV), (V) and (VII): 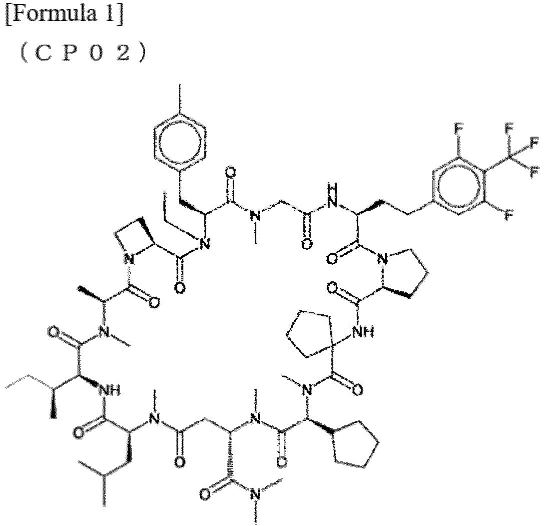
   (I) an anionic surfactant;
   (II) an amphoteric surfactant;
   (III) a nonionic surfactant;
   (IV) hydroxypropyl methyl cellulose or a derivative thereof;
   (V) a copolymer of a (meth)acrylic acid alkyl ester and at least one monomer selected from the group consisting of a (meth)acrylic acid, a (meth)acrylic acid alkyl ester, a (meth)acrylic acid alkylaminoalkyl ester and a (meth)acrylic acid ammonioalkyl ester; and
   (VII) a polyvinylcaprolactam-polyvinyl acetate-polyethylene glycol graft copolymer.
[8] A composition which comprises a peptide (CP02) represented by the following formula and one or more polymers selected from the group consisting of the following (IV), (V) and (VII), and is intended to be used in combination with one or more surfactants selected from the group consisting of the following (I) to (III): 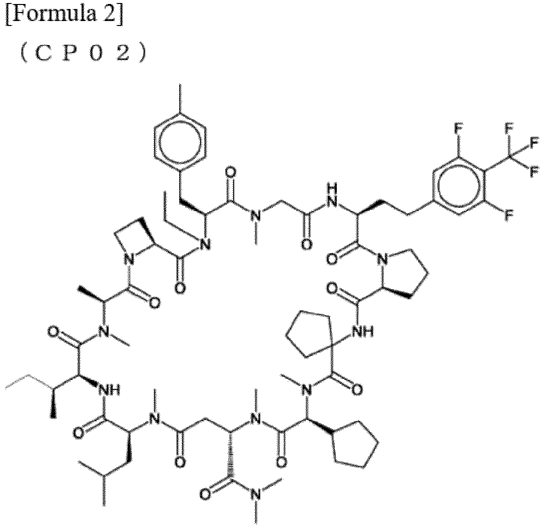
   (I) an anionic surfactant;
   (II) an amphoteric surfactant;
   (III) a nonionic surfactant;
   (IV) hydroxypropyl methyl cellulose or a derivative thereof;
   (V) a copolymer of a (meth)acrylic acid alkyl ester and at least one monomer selected from the group consisting of a (meth)acrylic acid, a (meth)acrylic acid alkyl ester, a (meth)acrylic acid alkylaminoalkyl ester and a (meth)acrylic acid ammonioalkyl ester; and
   (VII) a polyvinylcaprolactam-polyvinyl acetate-polyethylene glycol graft copolymer.
[9] A composition which one or more surfactants selected from the group consisting of the following (I) to (III), and is intended to be used in combination with a peptide (CP02) represented by the following formula and one or more polymers selected from the group consisting of the following (IV), (V) and (VII): 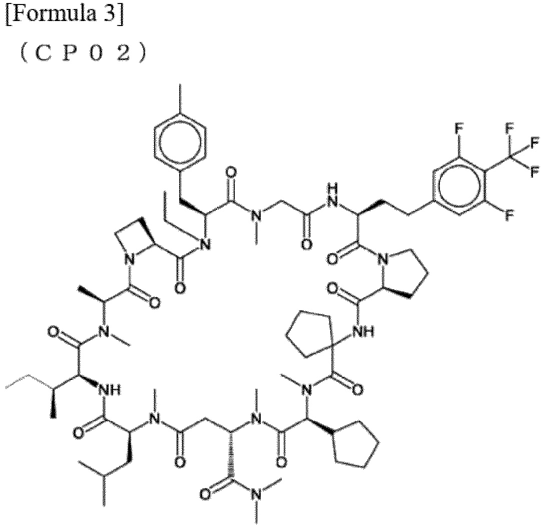
   (I) an anionic surfactant;
   (II) an amphoteric surfactant;
   (III) a nonionic surfactant;
   (IV) hydroxypropyl methyl cellulose or a derivative thereof;
   (V) a copolymer of a (meth)acrylic acid alkyl ester and at least one monomer selected from the group consisting of a (meth)acrylic acid, a (meth)acrylic acid alkyl ester, a (meth)acrylic acid alkylaminoalkyl ester and a (meth)acrylic acid ammonioalkyl ester; and
   (VII) a polyvinylcaprolactam-polyvinyl acetate-polyethylene glycol graft copolymer.
[10] The composition according to any one of [1] to [9], which is a pharmaceutical composition.
[11] The composition according to any one of [1] to [10], wherein a molecular weight of the peptide is 500 g/mol or more and 5,000 g/mol or less.
[12] The composition according to any one of [1] to [10], wherein the molecular weight of the peptide is 1,000 g/mol or more and 2,000 g/mol or less.
[13] The composition according to any one of [1] to [10], wherein the molecular weight of the peptide is 1,300 g/mol or more and 1,800 g/mol or less.
[14] The composition according to any one of [1] to [10], wherein the molecular weight of the peptide is 1,400 g/mol or more and 1,600 g/mol or less.
[15] The composition according to any one of [1] to [14], wherein the number of amino acid residues forming the peptide is 5 or more and 30 or less.
[16] The composition according to any one of [1] to [14], wherein the number of amino acid residues forming the peptide is 9 or more and 15 or less.
[17] The composition according to any one of [1] to [14], wherein the number of amino acid residues forming the peptide is 10 or more and 14 or less.
[18] The composition according to any one of [1] to [14], wherein the number of amino acid residues forming the peptide is 11 or more and 13 or less.
[19] The composition according to any one of [1] to [14], wherein the number of amino acid residues forming the peptide is 11.
[20] The composition according to any one of [1] to [19], wherein the peptide has a cyclic moiety.
[21] The composition according to [20], wherein the number of amino acid residues forming the cyclic moiety is 5 or more and 15 or less.
[22] The composition according to [20], wherein the number of amino acid residues forming the cyclic moiety is 9 or more and 15 or less.
[23] The composition according to [20], wherein the number of amino acid residues forming the cyclic moiety is 10 or more and 14 or less.
[24] The composition according to [20], wherein the number of amino acid residues forming the cyclic moiety is 11 or more and 13 or less.
[25] The composition according to [20], wherein the number of amino acid residues forming the cyclic moiety is 11.
[26] The composition according to any one of [20] to [25], wherein the cyclic moiety is composed of 28- to 55-membered ring.
[27] The composition according to any one of [20] to [25], wherein the cyclic moiety is composed of 31- to 46-membered ring.
[28] The composition according to any one of [20] to [25], wherein the cyclic moiety is composed of 34- to 43-membered ring.
[29] The composition according to any one of [20] to [25], wherein the cyclic moiety is composed of 34- to 37-membered ring.
[30] The composition according to any one of [20] to [25], wherein the cyclic moiety is composed of 34-membered ring.
[31] The composition according to any one of [1] to [30], wherein the number of N-substituted amino acid residues contained in the peptide is 1 or more.
[32] The composition according to any one of [1] to [30], wherein the number of N-substituted amino acid residues contained in the peptide is 3 or more.
[33] The composition according to any one of [1] to [30], wherein the number of N-substituted amino acid residues contained in the peptide is 4 or more.
[34] The composition according to any one of [1] to [30], wherein the number of N-substituted amino acid residues contained in the peptide is 5 or more.
[35] The composition according to any one of [31] to [34], wherein a nitrogen atom forming a main chain of the N-substituted amino acid is replaced with a C₁-C₆ alkyl, and the C₁-C₆ alkyl may form a ring with the nitrogen atom and a carbon atom bonded to the nitrogen atom.
[36] The composition according to any one of [31] to [34], wherein the N-substituted amino acid is an N-methylamino acid or an N-ethylamino acid.
[37] The composition according to any one of [31] to [34], wherein the N-substituted amino acid is an N-methylamino acid.
[38] The composition according to any one of [1] to [37], wherein the peptide contains at least one β-amino acid skeleton.
[39] The composition according to any one of [20] to [38], wherein the peptide contains at least one β-amino acid skeleton in the cyclic moiety.
[40] The composition according to any one of [1] to [39], wherein the number of N-unsubstituted amino acid residues contained in the peptide is 1 or more.
[41] The composition according to any one of [1] to [39], wherein the number of N-unsubstituted amino acid residues contained in the peptide is 2 or more.
[42] The composition according to any one of [1] to [39], wherein the number of N-unsubstituted amino acid residues contained in the peptide is 3 or more.
[43] The composition according to any one of [1] to [42], wherein the peptide does not have an indolyl group.
[44] The composition according to any one of [1] to [43], wherein the peptide does not have a substituted or unsubstituted hydroxyphenyl group.
[45] The composition according to any one of [1] to [42], wherein the peptide does not have an indolyl group and a substituted or unsubstituted hydroxyphenyl group.
[46] The composition according to any one of [1] to [45], wherein the number of aromatic rings contained in the peptide is 0 to 3.
[47] The composition according to any one of [1] to [45], wherein the number of aromatic rings contained in the peptide is 1 to 3.
[48] The composition according to any one of [1] to [47], wherein when the peptide has an acidic side chain, pKa of the acidic side chain is 3.5 to 10.
[49] The composition according to any one of [1] to [47], wherein when the peptide has an acidic side chain, pKa of the acidic side chain is 4.5 to 10.
[50] The composition according to any one of [1] to [47], wherein when the peptide has an acidic side chain, pKa of the acidic side chain is 5.0 to 10.
[51] The composition according to any one of [1] to [50], wherein when the peptide has a basic side chain, basic pKa of the basic side chain is 4.0 to 10.
[52] The composition according to any one of [1] to [50], wherein when the peptide has a basic side chain, basic pKa of the basic side chain is 4.0 to 9.0.
[53] The composition according to any one of [1] to [50], wherein when the peptide has a basic side chain, basic pKa of the basic side chain is 4.0 to 8.5.
[54] The composition according to any one of [1] to [50], wherein when the peptide has a basic side chain, basic pKa of the basic side chain is 4.0 to 7.5.
[55] The composition according to any one of [1] to [50], wherein when the peptide has a basic side chain, basic pKa of the basic side chain is 4.0 to 7.2.
[56] The composition according to any one of [1] to [55], wherein ClogP of the peptide is 4 or more and 25 or less.
[57] The composition according to any one of [1] to [55], wherein ClogP of the peptide is 6 or more and 23 or less.
[58] The composition according to any one of [1] to [55], wherein ClogP of the peptide is 8 or more and 21 or less.
[59] The composition according to any one of [1] to [55], wherein ClogP of the peptide is 9 or more and 20 or less.
[60] The composition according to any one of [1] to [59], wherein ClogP of the peptide is equal to or greater than that of the peptide (CP02) shown below. 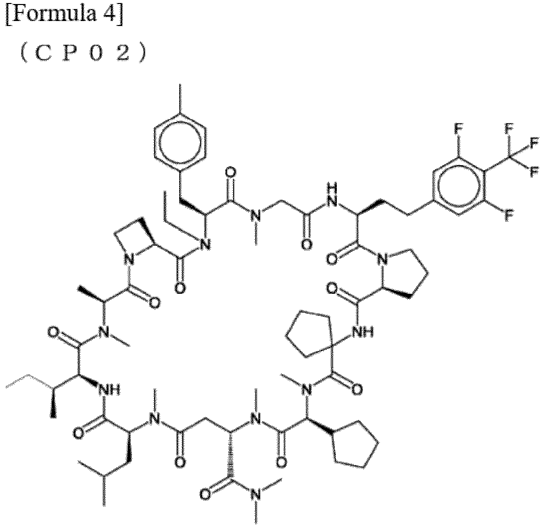
[61] The composition according to any one of [1] to [60], wherein a ClogP/number of amino acid residues of the peptide is 1.0 or more and 1.8 or less.
[62] The composition according to any one of [1] to [60], wherein a ClogP/number of amino acid residues of the peptide is 1.0 or more and 1.7 or less.
[63] The composition according to any one of [1] to [60], wherein a ClogP/number of amino acid residues of the peptide is 1.1 or more and 1.6 or less.
[64] The composition according to any one of [1] to [60], wherein a ClogP/number of amino acid residues of the peptide is 1.1 or more and 1.5 or less.
[65] The composition according to any one of [1] to [6] and [10] to [64], wherein the peptide is not any of the following peptides of (ECP1) to (ECP5). 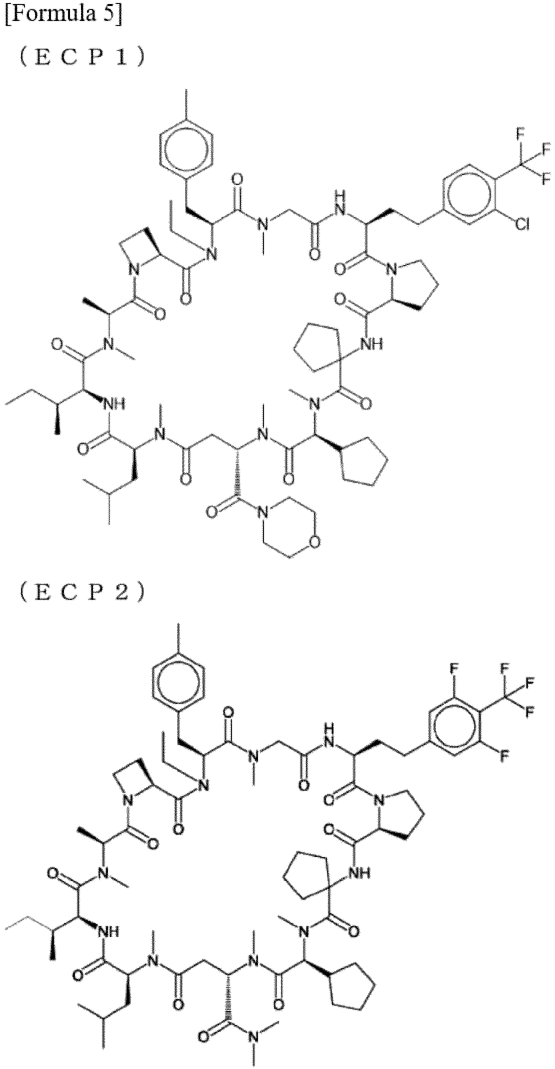 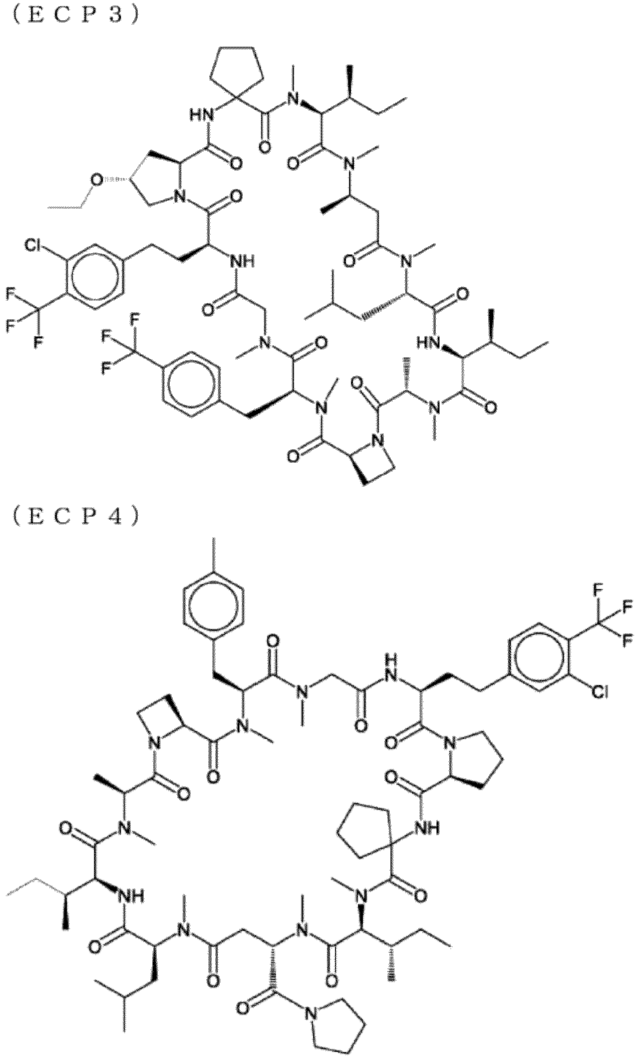 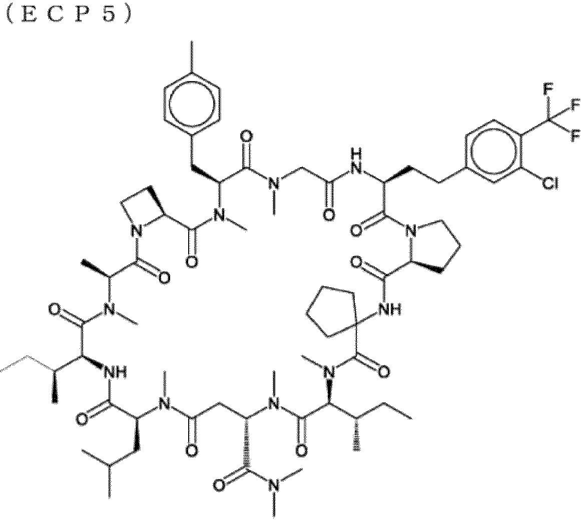
[66] The composition according to any one of [1] to [65], wherein the surfactant is an anionic surfactant.
[67] The composition according to any one of [1] to [65], wherein the surfactant is sodium lauryl sulfate.
[68] The composition according to any one of [1] to [65], wherein the surfactant is an amphoteric surfactant.
[69] The composition according to any one of [1] to [65], wherein the surfactant is acylcamitine.
[70] The composition according to any one of [1] to [65], wherein the surfactant is lauroyl-L-carnitine.
[71] The composition according to any one of [1] to [65], wherein the surfactant is a nonionic surfactant.
[72] The composition according to any one of [1] to [65], wherein the surfactant is a sucrose fatty acid ester.
[73] The composition according to any one of [1] to [65], wherein the surfactant is polyoxyethylene hydrogenated castor oil.
[74] The composition according to any one of [1] to [65], wherein the surfactant is D-α-tocopherol polyethylene glycol 1000 succinate.
[75] The composition according to any one of [1] to [74], wherein the polymer is a nonionic polymer.
[76] The composition according to any one of [1] to [74], wherein the polymer is an acidic polymer.
[77] The composition according to any one of [1] to [76], wherein the polymer is hydroxypropyl methyl cellulose or a derivative thereof.
[78] The composition according to any one of [1] to [76], wherein the polymer is hydroxypropyl methyl cellulose or an ester thereof.
[79] The composition according to any one of [1] to [76], wherein the polymer is one or more selected from the group consisting of hydroxypropyl methyl cellulose acetate succinate (HPMCAS), hydroxypropyl methyl cellulose phthalate (HPMCP), hydroxypropyl methyl acetate maleate and hydroxypropyl methyl trimellitate.
[80] The composition according to any one of [1] to [76], wherein the polymer is hydroxypropyl methyl cellulose acetate succinate (HPMCAS).
[81] The composition according to any one of [1] to [76], wherein the polymer is a copolymer of a (meth)acrylic acid alkyl ester and at least one monomer selected from the group consisting of a (meth)acrylic acid, a (meth)acrylic acid alkyl ester, a (meth)acrylic acid alkylaminoalkyl ester and a (meth)acrylic acid ammonioalkyl ester.
[82] The composition according to any one of [1] to [76], wherein the polymer is a copolymer of a (meth)acrylic acid alkyl ester and at least one monomer selected from the group consisting of (meth)acrylic acid, a (meth)acrylic acid alkyl ester and a (meth)acrylic acid alkylaminoalkyl ester.
[83] The composition according to any one of [1] to [76], wherein the polymer is a copolymer of a (meth)acrylic acid alkyl ester and at least one monomer selected from the group consisting of (meth)acrylic acid and a (meth)acrylic acid alkylaminoalkyl ester.
[84] The composition according to any one of [1] to [76], wherein the polymer is a copolymer of a (meth)acrylic acid alkyl ester and (meth)acrylic acid.
[85] The composition according to any one of [1] to [76], wherein the polymer is a copolymer of methyl methacrylate or ethyl acrylate and (meth)acrylic acid.
[86] The composition according to any one of [1] to [76], wherein the polymer is one or more selected from the group consisting of a copolymer of methyl methacrylate and methacrylic acid, and a copolymer of ethyl acrylate and methacrylic acid.
[87] The composition according to any one of [1] to [76], wherein the polymer is a copolymer of methyl methacrylate and methacrylic acid.
[88] The composition according to any one of [1] to [76], wherein the polymer is a polyvinylcaprolactam-polyvinyl acetate-polyethylene glycol graft copolymer.
[89] The composition according to any one of [1] to [65], wherein the surfactant is an anionic surfactant, and the polymer is hydroxypropyl methyl cellulose or a derivative thereof.
[90] The composition according to any one of [1] to [65], wherein the surfactant is sodium lauryl sulfate, and the polymer is hydroxypropyl methyl cellulose acetate succinate (HPMCAS).
[91] The composition according to any one of [1] to [65], wherein the surfactant is an anionic surfactant, and the polymer is a copolymer of a (meth)acrylic acid alkyl ester and at least one monomer selected from the group consisting of a (meth)acrylic acid, a (meth)acrylic acid alkyl ester, a (meth)acrylic acid alkylaminoalkyl ester and a (meth)acrylic acid ammonioalkyl ester.
[92] The composition according to any one of [1] to [65], wherein the surfactant is sodium lauryl sulfate, and the polymer is a copolymer of methyl methacrylate and methacrylic acid.
[93] The composition according to any one of [1] to [65], wherein the surfactant is an amphoteric surfactant, and the polymer is hydroxypropyl methyl cellulose or a derivative thereof.
[94] The composition according to any one of [1] to [65], wherein the surfactant is lauroyl-L-carnitine, and the polymer is hydroxypropyl methyl cellulose acetate succinate (HPMCAS).
[95] The composition according to any one of [1] to [65], wherein the surfactant is an amphoteric surfactant, and the polymer is a copolymer of a (meth)acrylic acid alkyl ester and at least one monomer selected from the group consisting of (meth)acrylic acid, a (meth)acrylic acid alkyl ester, a (meth)acrylic acid alkylaminoalkyl ester and a (meth)acrylic acid ammonioalkyl ester.
[96] The composition according to any one of [1] to [65], wherein the surfactant is lauroyl-L-carnitine, and the polymer is a copolymer of methyl methacrylate and methacrylic acid.
[97] The composition according to any one of [1] to [65], wherein the surfactant is a nonionic surfactant, and the polymer is hydroxypropyl methyl cellulose or a derivative thereof.
[98] The composition according to any one of [1] to [65], wherein the surfactant is a sucrose fatty acid ester, and the polymer is hydroxypropyl methyl cellulose acetate succinate (HPMCAS).
[99] The composition according to any one of [1] to [65], wherein the surfactant is polyoxyethylene hydrogenated castor oil, and the polymer is hydroxypropyl methyl cellulose acetate succinate (HPMCAS).
[100] The composition according to any one of [1] to [65], wherein the surfactant is D-α-tocopherol polyethylene glycol 1000 succinate, and the polymer is hydroxypropyl methyl cellulose acetate succinate (HPMCAS).
[101] The composition according to any one of [1] to [65], wherein the surfactant is an anionic surfactant, and the polymer is a polyvinylcaprolactam-polyvinyl acetate-polyethylene glycol graft copolymer.
[102] The composition according to any one of [1] to [65], wherein the surfactant is sodium lauryl sulfate, and the polymer is a polyvinylcaprolactam-polyvinyl acetate-polyethylene glycol graft copolymer.
[103] The composition according to any one of [1] to [102], wherein a ratio of a content of the surfactant to a content of the peptide on a weight basis is 0.1 or more and 40.0 or less.
[104] The composition according to any one of [1] to [102], wherein the ratio of the content of the surfactant to the content of the peptide on a weight basis is 0.1 or more and 15.0 or less.
[105] The composition according to any one of [1] to [102], wherein the ratio of the content of the surfactant to the content of the peptide on a weight basis is 0.3 or more and 6.0 or less.
[106] The composition according to any one of [1] to [102], wherein the ratio of the content of the surfactant to the content of the peptide on a weight basis is 0.5 or more and 3.0 or less.
[107] The composition according to any one of [1] to [106], wherein a ratio of a content of the polymer to the content of the peptide on a weight basis is 0.01 or more and 40.0 or less.
[108] The composition according to any one of [1] to [106], wherein the ratio of the content of the polymer to the content of the peptide on a weight basis is 0.01 or more and 15.0 or less.
[109] The composition according to any one of [1] to [106], wherein the ratio of the content of the polymer to the content of the peptide on a weight basis is 0.03 or more and 6.0 or less.
[110] The composition according to any one of [1] to [106], wherein the ratio of the content of the polymer to the content of the peptide on a weight basis is 0.1 or more and 3.0 or less.
[111] The composition according to any one of [1] to [110], wherein a ratio of a value of a solubility of the peptide when a composition composed of the peptide, the surfactant, the polymer and DMSO is dissolved in FaSSIF with a pH of 6.5 at 37°C to a total value of a solubility of the peptide when a composition composed of the peptide, the polymer and DMSO is dissolved in FaSSIF with a pH of 6.5 at 37°C and a solubility of the peptide when a composition composed of the peptide, the surfactant and DMSO is dissolved in FaSSIF with a pH of 6.5 at 37°C is more than 1.0.
[112] The composition according to any one of [1] to [110], wherein a ratio of a value of a solubility of the peptide when a composition composed of the peptide, the surfactant, the polymer and DMSO is dissolved in FaSSIF with a pH of 6.5 at 37°C to a total value of a solubility of the peptide when a composition composed of the peptide, the polymer and DMSO is dissolved in FaSSIF with a pH of 6.5 at 37°C and a solubility of the peptide when a composition composed of the peptide, the surfactant and DMSO is dissolved in FaSSIF with a pH of 6.5 at 37°C is more than 1.3.
[113] The composition according to any one of [1] to [110], wherein a ratio of a value of a solubility of the peptide when a composition composed of the peptide, the surfactant, the polymer and DMSO is dissolved in FaSSIF with a pH of 6.5 at 37°C to a total value of a solubility of the peptide when a composition composed of the peptide, the polymer and DMSO is dissolved in FaSSIF with a pH of 6.5 at 37°C and a solubility of the peptide when a composition composed of the peptide, the surfactant and DMSO is dissolved in FaSSIF with a pH of 6.5 at 37°C is more than 1.5.
[114] The composition according to any one of [1] to [113], wherein the peptide is an amorphous substance.
[115] The composition according to any one of [1] to [113], wherein the peptide is a crystalline substance.
[116] The composition according to any one of [1] to [113], which is for improvement of solubility of the peptide.
[117] The composition according to [116], wherein the improvement of solubility of the peptide is improvement of solubility of the peptide in both an amorphous state and a crystalline state.
[118] A composition comprising a peptide, which is used while coexisting with one or more surfactants selected from the group consisting of the following (I) to (III) and one or more polymers selected from the group consisting of the following (IV) to (VIII):
   (I) an anionic surfactant;
   (II) an amphoteric surfactant;
   (III) a nonionic surfactant;
   (IV) hydroxypropyl methyl cellulose or a derivative thereof;
   (V) a copolymer of a (meth)acrylic acid alkyl ester and at least one monomer selected from the group consisting of a (meth)acrylic acid, a (meth)acrylic acid alkyl ester, a (meth)acrylic acid alkylaminoalkyl ester and a (meth)acrylic acid ammonioalkyl ester;
   (VI) copovidone;
   (VII) a polyvinylcaprolactam-polyvinyl acetate-polyethylene glycol graft copolymer; and
   (VIII) polyvinylpyrrolidone.
[119] The composition according to any one of [1] to [118], wherein an active ingredient is the peptide.
[120] The composition according to any one of [7] to [119], wherein the peptide and the polymer form a solid dispersion.
[121] The composition according to any one of [4] to [6] and [10] to [120], wherein the solid dispersion is a spray-dried product.
[122] A method for producing a composition, comprising the step of combining a solid dispersion that is a spray-dried product of a mixture containing a peptide and one or more polymers selected from the group consisting of the following (IV) to (VIII) with one or more surfactants selected from the group consisting of the following (I) to (III) to obtain the composition:
   (I) an anionic surfactant;
   (II) an amphoteric surfactant;
   (III) a nonionic surfactant;
   (IV) hydroxypropyl methyl cellulose or a derivative thereof;
   (V) a copolymer of a (meth)acrylic acid alkyl ester and at least one monomer selected from the group consisting of a (meth)acrylic acid, a (meth)acrylic acid alkyl ester, a (meth)acrylic acid alkylaminoalkyl ester and a (meth)acrylic acid ammonioalkyl ester;
   (VI) copovidone;
   (VII) a polyvinylcaprolactam-polyvinyl acetate-polyethylene glycol graft copolymer; and
   (VIII) polyvinylpyrrolidone.
[123] The production method according to [122], further comprising the step of preparing a solid dispersion by removing a solvent from a solution containing the peptide and the polymer.
[124] The production method according to [122], further comprising the step of preparing a solid dispersion by spray-drying the mixture containing the peptide and the polymer.
[125] A method for producing a composition, comprising the step of combining a solid dispersion containing a peptide and one or more polymers selected from the group consisting of the following (IV) and (VII) with one or more surfactants selected from the group consisting of the following (I) to (III) to obtain the composition:
   (I) an anionic surfactant;
   (II) an amphoteric surfactant;
   (III) a nonionic surfactant;
   (IV) hydroxypropyl methyl cellulose or a derivative thereof; and
   (VII) a polyvinylcaprolactam-polyvinyl acetate-polyethylene glycol graft copolymer.
[126] A method for producing a composition, comprising the step of preparing a composition by combining a peptide (CP02) represented by the following formula, one or more surfactants selected from the group consisting of the following (I) to (III) and one or more polymers selected from the group consisting of the following (IV), (V) and (VII). 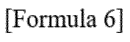 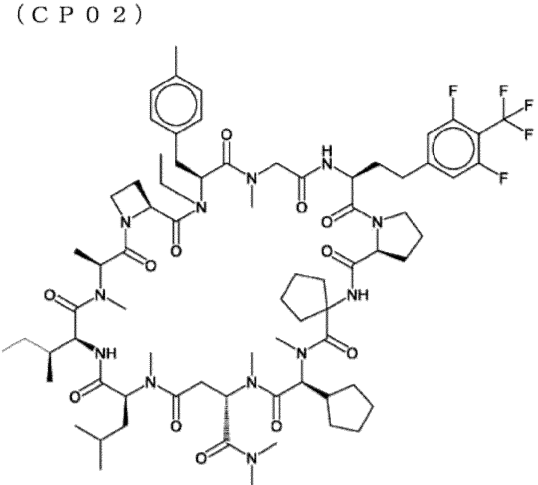
   (I) an anionic surfactant;
   (II) an amphoteric surfactant;
   (III) a nonionic surfactant;
   (IV) hydroxypropyl methyl cellulose or a derivative thereof;
   (V) a copolymer of a (meth)acrylic acid alkyl ester and at least one monomer selected from the group consisting of a (meth)acrylic acid, a (meth)acrylic acid alkyl ester, a (meth)acrylic acid alkylaminoalkyl ester and a (meth)acrylic acid ammonioalkyl ester; and
   (VII) a polyvinylcaprolactam-polyvinyl acetate-polyethylene glycol graft copolymer.
[127] A method for improving solubility of the peptide by combining a solid dispersion that is a spray-dried product of a mixture containing a peptide and one or more polymers selected from the group consisting of the following (IV) to (VIII) with one or more surfactants selected from the group consisting of the following (I) to (III):
   (I) an anionic surfactant;
   (II) an amphoteric surfactant;
   (III) a nonionic surfactant;
   (IV) hydroxypropyl methyl cellulose or a derivative thereof;
   (V) a copolymer of a (meth)acrylic acid alkyl ester and at least one monomer selected from the group consisting of a (meth)acrylic acid, a (meth)acrylic acid alkyl ester, a (meth)acrylic acid alkylaminoalkyl ester and a (meth)acrylic acid ammonioalkyl ester;
   (VI) copovidone;
   (VII) a polyvinylcaprolactam-polyvinyl acetate-polyethylene glycol graft copolymer; and
   (VIII) polyvinylpyrrolidone.
[128] A method for improving solubility of the peptide by combining a solid dispersion containing a peptide and one or more polymers selected from the group consisting of the following (IV) and (VII) with one or more surfactants selected from the group consisting of the following (I) to (III):
   (I) an anionic surfactant;
   (II) an amphoteric surfactant;
   (III) a nonionic surfactant;
   (IV) hydroxypropyl methyl cellulose or a derivative thereof; and
   (VII) a polyvinylcaprolactam-polyvinyl acetate-polyethylene glycol graft copolymer.
[129] A method for improving solubility of the peptide by combining a peptide (CP02) represented by the following formula, one or more surfactants selected from the group consisting of the following (I) to (III), and one or more polymers selected from the group consisting of the following (IV), (V) and (VII): 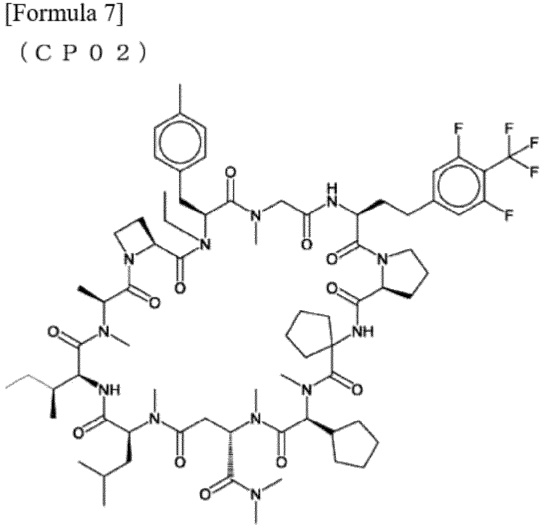
   (I) an anionic surfactant;
   (II) an amphoteric surfactant;
   (III) a nonionic surfactant;
   (IV) hydroxypropyl methyl cellulose or a derivative thereof;
   (V) a copolymer of a (meth)acrylic acid alkyl ester and at least one monomer selected from the group consisting of a (meth)acrylic acid, a (meth)acrylic acid alkyl ester, a (meth)acrylic acid alkylaminoalkyl ester and a (meth)acrylic acid ammonioalkyl ester; and
   (VII) a polyvinylcaprolactam-polyvinyl acetate-polyethylene glycol graft copolymer.
[130] The method for improving solubility of the peptide according to [127] to [129], wherein the improvement of solubility of the peptide is improvement of solubility of the peptide in both an amorphous state and a crystalline state.
[131] Use or application of one or more surfactants selected from the group consisting of the following (I) to (III), as an agent for improving solubility of a peptide present in a solid dispersion that is a spray-dried product of a mixture containing the peptide and one or more polymers selected from the group consisting of the following (IV) to (VIII):
   (I) an anionic surfactant;
   (II) an amphoteric surfactant;
   (III) a nonionic surfactant;
   (IV) hydroxypropyl methyl cellulose or a derivative thereof;
   (V) a copolymer of a (meth)acrylic acid alkyl ester and at least one monomer selected from the group consisting of a (meth)acrylic acid, a (meth)acrylic acid alkyl ester, a (meth)acrylic acid alkylaminoalkyl ester and a (meth)acrylic acid ammonioalkyl ester;
   (VI) copovidone;
   (VII) a polyvinylcaprolactam-polyvinyl acetate-polyethylene glycol graft copolymer; and
   (VIII) polyvinylpyrrolidone.
[132] Use or application of one or more surfactants selected from the group consisting of the following (I) to (III), as an agent for improving solubility of a peptide present in a solid dispersion containing the peptide and one or more polymers selected from the group consisting of the following (IV) and (VIII):
   (I) an anionic surfactant;
   (II) an amphoteric surfactant;
   (III) a nonionic surfactant;
   (IV) hydroxypropyl methyl cellulose or a derivative thereof; and
   (VII) a polyvinylcaprolactam-polyvinyl acetate-polyethylene glycol graft copolymer.
[133] Use or application of a combination of one or more surfactants selected from the group consisting of the following (I) to (III) and one or more polymers selected from the group consisting of the following (IV), (V) and (VII), as an agent for improving solubility of a peptide (CP02) represented by the following formula: 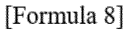 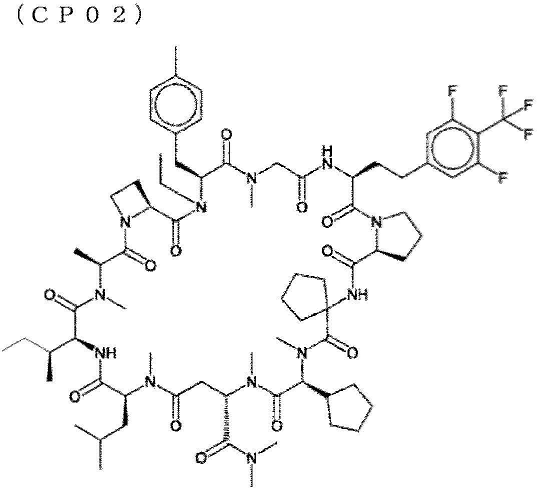
   (I) an anionic surfactant;
   (II) an amphoteric surfactant;
   (III) a nonionic surfactant;
   (IV) hydroxypropyl methyl cellulose or a derivative thereof;
   (V) a copolymer of a (meth)acrylic acid alkyl ester and at least one monomer selected from the group consisting of a (meth)acrylic acid, a (meth)acrylic acid alkyl ester, a (meth)acrylic acid alkylaminoalkyl ester and a (meth)acrylic acid ammonicalkyl ester; and
   (VII) a polyvinylcaprolactam-polyvinyl acetate-polyethylene glycol graft copolymer.
[134] The use or application according to [131] to [133], wherein the improvement of solubility of the peptide is improvement of solubility of the peptide in both an amorphous state and a crystalline state.

### [Advantageous Effects of Invention]

According to the present invention, a composition containing a peptide, which has enhanced solubility of the peptide, can be provided.

According to the present invention, the composition containing a peptide, in which solubility of the peptide is enhanced without relying on the crystallization suppressing action of a polymer, can be provided.

According to the present invention, a composition containing a peptide, in which a surfactant and a polymer exhibits an improved synergistic solubilizing effect to enhance solubility of the peptide, can be provided.

### [Description of Embodiments]

Hereinafter, the embodiments for carrying out the present invention are described in detail. However, the present invention is not limited by the embodiments given below.

The term "one or more" as used herein means the number of 1 or 2 or larger. When the term "one or more" is used in the context associated with a substituent of a group, the term means a number from 1 to the maximum number of substituents acceptable by the group. Specific examples of the term "one or more" include 1, 2, 3, 4, 5, 6, 7, 8, 9, 10, and/or larger numbers.

As used herein, "to" indicating a range means that values at both ends thereof are included, and for example, "A to B" means a range of A or more and B or less.

The term "about" as used herein, when used in combination with a numeric value, means the value range of +10% and -10% of the numeric value.

In the present invention, the meaning of the term "and/or" includes any combination in which "and" and "or" are appropriately combined. Specifically, for example, the term "A, B, and/or C" includes the following seven variations: (i) A, (ii) B, (iii) C, (iv) A and B, (v) A and C, (vi) B and C, and (vii) A, B, and C.

The term "DMSO" as used herein means dimethyl sulfoxide.

The term "wt/vol%" as used herein represents weight% by volume (weight/volume%).

A composition according to the present embodiment contains a peptide, one or more surfactants selected from the group consisting of the following (I) to (III), and one or more polymers selected from the group consisting of the following (IV) to (VIII). In an aspect, the composition according to the present embodiment contains a solid dispersion that is a spray-dried product of a mixture containing a peptide and one or more polymers selected from the group consisting of the following (IV) to (VIII), and one or more surfactants selected from the group consisting of the following (I) to (III). In an aspect, the composition according to the present embodiment contains a solid dispersion containing a peptide and one or more polymers selected from the group consisting of the following (IV) and (VII), and one or more surfactants selected from the group consisting of the following (I) to (III). In an aspect, the composition according to the present embodiment contains a peptide (CP02) represented by the following formula: 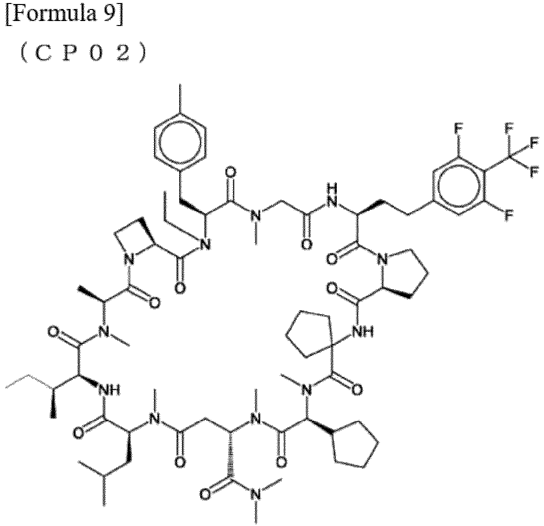 and one or more surfactants selected from the group consisting of the following (I) to (III), and one or more polymers selected from the group consisting of the following (IV), (V) and (VII). The composition according to the present embodiment is preferably a pharmaceutical composition.
(I) An anionic surfactant;
(II) an amphoteric surfactant;
(III) a nonionic surfactant;
(IV) hydroxypropyl methyl cellulose or a derivative thereof;
(V) a copolymer of a (meth)acrylic acid alkyl ester and at least one monomer selected from the group consisting of a (meth)acrylic acid, a (meth)acrylic acid alkyl ester, a (meth)acrylic acid alkylaminoalkyl ester and a (meth)acrylic acid ammonioalkyl ester;
(VI) copovidone;
(VII) a polyvinylcaprolactam-polyvinyl acetate-polyethylene glycol graft copolymer; and
(VIII) polyvinylpyrrolidone.

### [Peptide]

The "peptide" as used herein is not particularly limited as long as it is a peptide formed from natural amino acids and/or non-natural amino acids linked via an amide bond or an ester bond.

The molecular weight of the peptide in the present embodiment is not particularly limited. It may be, for example, 500 g/mol or more, 550 g/mol or more, 600 g/mol or more, 650 g/mol or more, 700 g/mol or more, 750 g/mol or more, 800 g/mol or more, 850 g/mol or more, 900 g/mol or more, 950 g/mol or more, 1,000 g/mol or more, 1,100 g/mol or more, 1,200 g/mol or more, 1,300 g/mol or more, or 1,400 g/mol or more, and may be 5,000 g/mol or less, 4,000 g/mol or less, 3,000 g/mol or less, 2,500 g/mol or less, 2,000 g/mol or less, 1,900 g/mol or less, 1,800 g/mol or less, 1,700 g/mol or less, or 1,600 g/mol or less. The molecular weight of the peptide in the present embodiment is not particularly limited, and may be, for example, 500 g/mol or more and 5,000 g/mol or less, 700 g/mol or more and 4,000 g/mol or less, 800 g/mol or more and 3,000 g/mol or less, 900 g/mol or more and 2,500 g/mol or less, 1,000 g/mol or more and 2,000 g/mol or less, 1,200 g/mol or more and 1,900 g/mol or less, 1,300 g/mol or more and 1,800 g/mol or less, or 1,400 g/mol or more and 1,600 g/mol or less. The molecular weight of the peptide in the present embodiment is not particularly limited, and is, for example, 500 g/mol or more and 5,000 g/mol or less, preferably 1,000 g/mol or more and 2,000 g/mol or less, more preferably 1,300 g/mol or more and 1,800 g/mol or less, and most preferably 1,400 g/mol or more and 1,600 g/mol or less. The molecular weight as used herein means the sum of the atomic weights of the atoms constituting the compound molecule (unit: "g/mol"), and is obtained by calculating the sum of the atomic weights of the atoms included in the molecular structure formula (unit: "g/mol"). The unit of molecular weight is sometimes omitted herein.

As used herein, the term "number of amino acids" or "number of amino acid residues" refers to the number of amino acid residues (amino acid units) constituting a peptide, and means the number of amino acid units generated upon cleavage of amide bonds, ester bonds, and bonds of cyclized moieties that link the amino acids. For example, the number of amino acids or the number of amino acid residues of the cyclic peptide in which the cyclic moiety is composed of 10 amino acid residues and the linear moiety is composed of 1 amino acid residue is 11.

The number of amino acid residues forming the peptide in the present embodiment is not particularly limited. It may be, for example, 5 or more, 6 or more, 7 or more, 8 or more, 9 or more, 10 or more, or 11 or more, and may be 30 or less, 25 or less, 20 or less, 17 or less, 15 or less, 14 or less, 13 or less, 12 or less, or 11 or less. The number of amino acid residues forming the peptide in the present embodiment is not particularly limited, and may be, for example, 5 or more and 30 or less, 6 or more and 25 or less, 7 or more and 20 or less, 8 or more and 17 or less, 9 or more and 15 or less, 10 or more and 14 or less, 11 or more and 13 or less, 11 or more and 12 or less, or 11. The number of amino acid residues forming the peptide in the present embodiment is not particularly limited, and is, for example, 5 or more and 30 or less, preferably 9 or more and 15 or less, more preferably 11 or more and 13 or less, and most preferably 11.

As used herein, the term "amino acid" includes a natural amino acid and a non-natural amino acid (sometimes also referred to as an amino acid derivative). Also, as used herein, the term "amino acid residue" includes a natural amino acid residue and a non-natural amino acid (amino acid derivative) residue.

Natural amino acids refer to glycine (Gly), L-alanine (Ala), L-serine (Ser), L-threonine (Thr), L-valine (Val), L-leucine (Leu), L-isoleucine (Ile), L-phenylalanine (Phe), L-tyrosine (Tyr), L-tryptophan (Trp), L-histidine (His), L-glutamic acid (Glu), L-aspartic acid (Asp), L-glutamine (Gln), L-asparagine (Asn), L-cysteine (Cys), L-methionine (Met), L-lysine (Lys), L-arginine (Arg), and L-proline (Pro).

Non-natural amino acids (amino acid derivatives) are not particularly limited, and examples thereof include a β-amino acid, a D-type amino acid, an N-substituted amino acid (excluding Pro), an α,α-disubstituted amino acid, an amino acid having a side chain different from that of natural amino acids, and a hydroxycarboxylic acid. As used herein, non-natural N-substituted amino acids mean N-substituted amino acids other than Pro.

As the amino acid as used herein, any steric configuration is acceptable. The selection of a side chain of the amino acid is not particularly restricted, and the side chain is freely selected from, in addition to a hydrogen atom, an alkyl group, an alkenyl group, an alkynyl group, an aryl group, a heteroaryl group, an aralkyl group, a heteroaralkyl group, a cycloalkyl group, and a spiro-bonded cycloalkyl group. Each of the side chains may have a substituent. The substituent is also not limited, and one or two or more substituents may be freely selected independently from any substituents including, for example, a halogen atom, an O atom, an S atom, an N atom, a B atom, a Sᵢ atom, or a P atom. That is, examples of the side chain include an alkyl group, an alkoxy group, an alkenyl group, an alkynyl group, an aryl group, a heteroaryl group, an aralkyl group, or a cycloalkyl group, which may be substituted, oxo, aminocarbonyl, and a halogen atom. The amino acid according to one embodiment may be a compound having a carboxy group and an amino group in the same molecule, and even in this case, imino acids such as L-proline and hydroxyproline are also included in the amino acid.

As used herein, the term "alkyl" is a monovalent group induced by the removal of any one hydrogen atom from aliphatic hydrocarbon, and has a subset of a hydrocarbyl or hydrocarbon group structure containing hydrogen and carbon atoms without containing a heteroatom (which refers to an atom other than carbon and hydrogen atoms) or an unsaturated carbon-carbon bond in the skeleton. The alkyl includes not only a linear form but also a branched form. Preferred examples of the alkyl include alkyl having 1 to 20 carbon atoms (C₁-C₂₀; hereinafter, "Cₚ-C_{q}" means that the number of carbon atoms is p to q), preferably C₁-C₁₀ alkyl, and more preferably C₁-C₆ alkyl. Specific examples of the alkyl include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl.

The term "alkynyl" as used herein is a monovalent group having at least one triple bond (two adjacent SP carbon atoms). The alkynyl includes not only a linear form but a branched form. Preferred examples of the alkynyl include C₂-C₁₀ alkynyl, and more preferably C₂-C₆ alkynyl. Specific examples thereof include ethynyl, 1-propynyl, propargyl, 3-butynyl, pentynyl, hexynyl, 3-phenyl-2-propynyl, 3-(2'-fluorophenyl)-2-propynyl, 2-hydroxy-2-propynyl, 3-(3-fluorophenyl)-2-propynyl, and 3-methyl-(5-phenyl)-4-pentynyl.

The term "alkenyl" as used herein is a monovalent group having at least one double bond (two adjacent SP2 carbon atoms). Depending on the conformation of the double bond and a substituent (if present), the geometric morphology of the double bond can assume entgegen (E) or zusammen (Z) and cis or trans conformations. The alkenyl includes not only a linear form but a branched form. Preferred examples of the alkenyl include C₂-C₁₀ alkenyl, and more preferred examples thereof include C₂-C₆ alkenyl. Specific examples thereof include vinyl, allyl, 1-propenyl, 2-propenyl, 1-butenyl, 2-butenyl (including cis and trans), 3-butenyl, pentenyl, 3-methyl-2-butenyl, and hexenyl.

The term "aryl" as used herein means a monovalent aromatic hydrocarbon ring and an aromatic hydrocarbon ring group. Preferred examples of the aryl include C₆-C₁₀ aryl. Specific examples of the aryl include phenyl and naphthyl (for example, 1-naphthyl and 2-naphthyl).

The term "heteroaryl" as used herein means an aromatic cyclic monovalent group containing 1 to 5 heteroatoms in addition to a carbon atom, and an aromatic heterocyclic group. The ring may be a monocyclic ring or a condensed ring with another ring, and may be partially saturated. The number of atoms constituting the ring of heteroaryl is preferably 5 to 10 (5- to 10-membered heteroaryl), and more preferably 5 to 7 (5- to 7-membered heteroaryl). Specific examples of the heteroaryl include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzoxadiazolyl, benzimidazolyl, benzotriazolyl, indolyl, isoindolyl, indazolyl, azaindolyl, quinolyl, isoquinolyl, cinnolinyl, quinazolinyl, quinoxalinyl, benzodioxolyl, indolizinyl, imidazopyridyl, pyrazolopyridyl, imidazopyridyl, triazolopyridyl, pyrrolopyrazinyl, and flopyridyl.

The term "aralkyl (arylalkyl)" as used herein means a group in which at least one hydrogen atom of the "alkyl" as defined above is replaced with the "aryl" as defined above. As the aralkyl, C₇-C₁₄ aralkyl is preferred, and C₇-C₁₀ aralkyl is more preferred. Specific examples of the aralkyl include benzyl, phenethyl, and 3-phenylpropyl.

The term "heteroaralkyl" as used herein means a group in which at least one hydrogen atom of the "alkyl" as defined above is replaced with the "heteroaryl" as defined above. As the heteroaralkyl, 5- to 10-membered heteroaryl-C₁-C₆ alkyl is preferred, and 5- to 10-membered heteroaryl-C₁-C₂ alkyl is more preferred. Specific examples of the heteroaralkyl include 3-thienylmethyl, 4-thiazolylmethyl, 2-pyridylmethyl, 3-pyridylmethyl, 4-pyridylmethyl, 2-(2-pyridyl)ethyl, 2-(3-pyridyl)ethyl, 2-(4-pyridyl)ethyl, 2-(6-quinolyl)ethyl, 2-(7-quinolyl)ethyl, 2-(6-indolyl)ethyl, 2-(5-indolyl)ethyl, and 2-(5-benzofuranyl)ethyl.

In the present specification, the "cycloalkyl" means a saturated or partially saturated cyclic monovalent aliphatic hydrocarbon group and includes a monocyclic ring, a bicyclo ring, and a spiro ring. Preferred examples of the cycloalkyl include C₃-C₈ cycloalkyl. Specific examples thereof include cyclopropyl, cyclobutyl, cyclopentyl, cyclohexyl, cycloheptyl, cyclooctyl, bicyclo[2.2.1]heptyl, and spiro[3.3]heptyl.

In the present specification, the "amino" means -NH₂ in the narrow sense and means - NRR' in the broad sense. In this context, R and R' are each independently selected from hydrogen, alkyl, alkenyl, alkynyl, cycloalkyl, heterocyclyl, aryl, and heteroaryl, or R and R' form a ring together with the nitrogen atom bonded thereto. Preferred examples of the amino include -NH₂, mono-C₁-C₆ alkylamino, di-C₁-C₆ alkylamino, and 4- to 8-membered cyclic amino.

The term "monoalkylamino" as used herein means a group of the "amino" as defined above in which R is hydrogen and R' is the "alkyl" as defined above. Preferred examples of the monoalkylamino include mono-C₁-C₆ alkylamino. Specific examples of the monoalkylamino include methylamino, ethylamino, n-propylamino, i-propylamino, n-butylamino, s-butylamino, and t-butylamino.

The term "dialkylamino" as used herein means a group of the "amino" as defined above in which R and R' are each independently the "alkyl" as defined above. Preferred examples of the dialkylamino include di-C₁-C₆ alkylamino. Specific examples of the dialkylamino include dimethylamino and diethylamino.

The term "alkylsulfonylamino" as used herein means a group in which sulfonyl is bonded to the "amino" as defined above. Preferred examples thereof include C₁-C₆ alkylsulfonyl-NH- and (C₁-C₆ alkylsulfonyl-)₂-N-. Specific examples of the aminoalkylsulfonyl include methylsulfonylamino, ethylsulfonylamino, bis(methylsulfonyl)amino, and bis(ethylsulfonyl)amino.

The term "aminocarbonyl" as used herein means a carbonyl group to which the "amino" as defined above is bonded. Preferred examples of the aminocarbonyl include -CONH₂, mono-C₁-C₆ alkylaminocarbonyl, di-C₁-C₆ alkylaminocarbonyl, and 4- to 8-membered cyclic aminocarbonyl. Specific examples of the aminocarbonyl include -CONH₂, dimethylaminocarbonyl, 1-azetidinylcarbonyl, 1-pyrrolidinylcarbonyl, 1-piperidinylcarbonyl, 1-piperazinylcarbonyl, 4-morpholinylcarbonyl, 3-oxazolidinylcarbonyl, 1,1-dioxidothiomorpholinyl-4-ylcarbonyl, and 3-oxa-8-azabicyclo[3.2.1]octan-8-ylcarbonyl.

Examples of halogen-derived substituents include fluoro (-F), chloro (-Cl), bromo (-Br), and iodo (-I).

Examples of O-atom-derived substituents include hydroxy (-OH), oxy (-OR), carbonyl (-C(=O)-R), carboxy (-CO₂H), oxycarbonyl(-C(=O)-OR), carbonyloxy (-O-C(=O)-R), thiocarbonyl (-C(=O)-SR), a carbonylthio group (-S-C(=O)-R), aminocarbonyl (-C(=O)-NHR), carbonylamino (-NH-C(=O)-R), oxycarbonylamino (-NH-C(=O)-OR), sulfonylamino (-NH-SO₂-R), aminosulfonyl (-SO₂-NHR), sulfamoylamino (-NH-SO₂-NHR), thiocarboxy (-C(=O)-SH), and carboxylcarbonyl (-C(=O)-CO₂H).

Examples of oxy (-OR) include alkoxy, cycloalkoxy, alkenyloxy, alkynyloxy, aryloxy, heteroaryloxy, and aralkyloxy.

Examples of carbonyl (-C(=O)-R) include formyl (-C(=O)-H), alkylcarbonyl, cycloalkylcarbonyl, alkenylcarbonyl, alkynylcarbonyl, arylcarbonyl, heteroarylcarbonyl, and aralkylcarbonyl.

Examples of oxycarbonyl (-C(=O)-OR) include alkyloxycarbonyl, cycloalkyloxycarbonyl, alkenyloxycarbonyl, alkynyloxycarbonyl, aryloxycarbonyl, heteroaryloxycarbonyl, and aralkyloxycarbonyl.

Examples of carbonyloxy (-O-C(=O)-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of thiocarbonyl (-C(=O)-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of carbonylthio (-S-C(=O)-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of aminocarbonyl (-C(=O)-NHR) include alkylaminocarbonyl, cycloalkylaminocarbonyl, alkenylaminocarbonyl, alkynylaminocarbonyl, arylaminocarbonyl, heteroarylaminocarbonyl, and aralkylaminocarbonyl. In addition to these, examples include compounds in which an H atom bonded to the N atom in -C(=O)-NHR is further replaced with alkyl, cycloalkyl, alkenyl, alkynyl, aryl, heteroaryl, or aralkyl.

Examples of carbonylamino (-NH-C(=O)-R) include alkylcarbonylamino, cycloalkylcarbonylamino, alkenylcarbonylamino, alkynylcarbonylamino, arylcarbonylamino, heteroarylcarbonylamino, and aralkylcarbonylamino. In addition to these, examples include compounds in which an H atom bonded to the N atom in -NH-C(=O)-R is further replaced with alkyl, cycloalkyl, alkenyl, alkynyl, aryl, heteroaryl, or aralkyl.

Examples of oxycarbonylamino (-NH-C(=O)-OR) include alkoxycarbonylamino, cycloalkoxycarbonylamino, alkenyloxycarbonylamino, alkynyloxycarbonylamino, aryloxycarbonylamino, heteroaryloxycarbonylamino, and aralkyloxycarbonylamino. In addition to these, examples include compounds in which an H atom bonded to the N atom in - NH-C(=O)-OR is further replaced with alkyl, cycloalkyl, alkenyl, alkynyl, aryl, heteroaryl, or aralkyl.

Examples of sulfonylamino (-NH-SO₂-R) include alkylsulfonylamino, cycloalkylsulfonylamino, alkenylsulfonylamino, alkynylsulfonylamino, arylsulfonylamino, heteroarylsulfonylamino, and aralkylsulfonylamino. In addition to these, examples include compounds in which an H atom bonded to the N atom in -NH-SO₂-R is further replaced with alkyl, cycloalkyl, alkenyl, alkynyl, aryl, heteroaryl, or aralkyl.

Examples of aminosulfonyl (-SO₂-NHR) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. In addition to these, examples include compounds in which an H atom bonded to the N atom in -SO₂-NHR is further replaced with alkyl, cycloalkyl, alkenyl, alkynyl, aryl, heteroaryl, or aralkyl.

Examples of sulfamoylamino (-NH-SO₂-NHR) include alkylsulfamoylamino, cycloalkylsulfamoylamino, alkenylsulfamoylamino, alkynylsulfamoylamino, arylsulfamoylamino, heteroarylsulfamoylamino, and aralkylsulfamoylamino. Further, the two H atoms bonded to the N atom in -NH-SO₂-NHR may be replaced with substituents independently selected from the group consisting of alkyl, cycloalkyl, alkenyl, alkynyl, aryl, heteroaryl, and aralkyl, and the two substituents may form a ring.

Examples of S atom-derived substituents include thiol (-SH), thio (-S-R), sulfinyl (-S(=O)-R), sulfonyl (-S(O)₂-R), sulfo (-SO₃H), and pentafluorosulfanyl (-SF₅).

Examples of thio (-S-R) include alkylthio, cycloalkylthio, alkenylthio, alkynylthio, arylthio, heteroarylthio, and aralkylthio.

Examples of sulfinyl (-S(=O)-R) include alkylsulfinyl, cycloalkylsulfinyl, alkenylsulfinyl, alkynylsulfinyl, arylsulfinyl, heteroarylsulfinyl, and aralkylsulfinyl.

Examples of sulfonyl (-S(O)₂-R) include alkylsulfonyl, cycloalkylsulfonyl, alkenylsulfonyl, alkynylsulfonyl, arylsulfonyl, heteroarylsulfonyl, and aralkylsulfonyl.

Examples of N-atom-derived substituents include azido (-N₃, also referred to as "azido group"), cyano (-CN), primary amino (-NH₂), secondary amino (-NH-R), tertiary amino (-NR(R')), amidino (-C(=NH)-NH₂), substituted amidino (-C(=NR)-NR'R"), guanidino (-NH-C(=NH)-NH₂), substituted guanidino (-NR-C(=NR"')-NR'R"), and aminocarbonylamino (-NR-CO-NR'R").

Examples of secondary amino (-NH-R) include alkylamino, cycloalkylamino, alkenylamino, alkynylamino, arylamino, heteroarylamino, and aralkylamino.

Examples of tertiary amino (-NR(R')) include an amino group having any two substituents each independently selected from alkyl, cycloalkyl, alkenyl, alkynyl, aryl, heteroaryl, aralkyl, and the like, such as alkyl(aralkyl)amino, and the two substituents may form a ring.

Examples of substituted amidino (-C(=NR)-NR'R") include groups in which the three substituents R, R', and R" on the N atom are each independently selected from alkyl, cycloalkyl, alkenyl, alkynyl, aryl, heteroaryl, and aralkyl, such as alkyl(aralkyl)(aryl)amidino.

Examples of substituted guanidino (-NR-C(=NR‴)-NR'R") include a group in which R, R', R", and R‴ are each independently selected from alkyl, cycloalkyl, alkenyl, alkynyl, aryl, heteroaryl, and aralkyl, and a group in which they form a ring.

Examples of aminocarbonylamino (-NR-CO-NR'R") include a group in which R, R', and R" are each independently selected from a hydrogen atom, alkyl, cycloalkyl, alkenyl, alkynyl, aryl, heteroaryl, and aralkyl, and a group in which they form a ring.

Examples of B-atom-derived substituents include boryl (-BR(R")), and dioxyboryl (-B(OR)(OR')). The two substituents R and R' may be groups each independently selected from alkyl, cycloalkyl, alkenyl, alkynyl, aryl, heteroaryl, aralkyl, or the like, or may be a group in which they form a ring. Specific examples include a cyclic boryl group, and more specifically include a pinacolate boryl group, a neopentanediolate boryl group, and a catecholate boryl group.

The amino group of the main chain of the amino acid may be unsubstituted (-NH₂) or substituted (that is, -NHR). R represents, for example, an alkyl group, an alkenyl group, an alkynyl group, an aryl group, a heteroaryl group, an aralkyl group, or a cycloalkyl group, which is optionally substituted, or a carbon chain attached to the N atom and a carbon atom at position α may form a ring, like proline.

The peptide in the present embodiment may have a cyclic moiety, and preferably has a cyclic moiety. In other words, the peptide in the present embodiment may be a cyclic peptide, and is preferably a cyclic peptide. The term "cyclic peptide" as used herein is a peptide having a cyclic structure composed of 4 or more amino acid residues. As an aspect of cyclization of the cyclic peptide, it may take any form, such as cyclization with a carbon-nitrogen bond such as an amide bond, cyclization with a carbon-oxygen bond such as an ester bond or ether bond, cyclization with a carbon-sulfur bond such as a thioether bond, cyclization with a carbon-carbon bond, or cyclization by heterocyclic construction. Among these, cyclization via a covalent bond such as an amide bond, a carbon-sulfur bond, or a carbon-carbon bond is preferred. Cyclization with an amide bond is more preferred, and the position of the carboxyl group or amino group used for cyclization may be on the main chain or on a side chain. Most preferred is cyclization via an amide bond between a carboxyl group on a side chain and the amino group of the main chain at the N-terminus.

In the present specification, the "heterocyclic ring" means a nonaromatic heterocyclic ring containing preferably 1 to 5, more preferably 1 to 3 heteroatoms among the atoms constituting the ring. The heterocyclic ring may have a double and/or triple bond in the ring. A carbon atom in the ring may form carbonyl through oxidation, and the ring may be a monocyclic ring, a condensed ring, or a spiro ring. The number of atoms constituting the ring of the heterocyclic ring is preferably 3 to 12 (3- to 12-membered heterocyclic ring), and more preferably 4 to 10 (4-to 10-membered heterocyclic ring). Specific examples of the heterocyclic ring include an azetidine ring, an oxetane ring, a tetrahydrofuran ring, a tetrahydropyran ring, a morpholine ring, a thiomorpholine ring, a pyrrolidine ring, a 4-oxopyrrolidine ring, a piperidine ring, a 4-oxopiperidine ring, a piperazine ring, a pyrazolidine ring, an imidazolidine ring, an oxazolidine ring, an isoxazolidine ring, a thiazolidine ring, an isothiazolidine ring, a thiadiazolidine ring, an oxazolidone ring, a dioxolane ring, a dioxane ring, a thietane ring, an octahydroindole ring, a 6,7-dihydro-pyrrolo[1,2-a]imidazole ring, an azocane ring, a 4,5,6,7-tetrahydropyrazolo[1,5-a]pyrazine ring, an azepane ring, a dioxepane ring, a 5,9-dioxaspiro[3.5]nonane ring, or a ring in which one or more single bonds in any of these saturated heterocyclic rings are replaced with double bonds or triple bonds.

The term "cyclization" of a peptide means formation of a cyclic moiety containing 4 or more amino acid residues. The method for converting a chain peptide to a cyclic peptide can be performed by carrying out an intramolecular bond formation reaction by the method described in Comprehensive Organic Transformations, A Guide to Functional Group Preparations, 3rd Edition (authored by R. C. Larock), March's Advanced Organic Chemistry: Reactions, Mechanisms, and Structure, 7th Edition (authored by M. B. Smith and J. March), or the like. It is also possible to further carry out a functional group conversion reaction after the bond formation reaction. Examples of the bond at the cyclization site of the cyclic peptide include a C(O)-N bond formed from a carboxylic acid and an amine, a C-O-C bond, a C(O)-O bond, and a C(S)-O bond via an oxygen atom, a C(O)-S bond, a C(S)-S bond, a C-S-S-C bond, a C-S-C bond, a C-S(O)-C bond, and a C-S(O₂)-C bond via a sulfur atom, and a C-N-C bond, a C=N-C bond, an N-C(O)-N bond, an N-C(S)N bond, and a C(S)-N bond via a nitrogen atom. Further examples thereof include a C-C bond formed by a coupling reaction catalyzed by a transition metal, such as the Suzuki reaction, the Heck reaction, and the Sonogashira reaction. Examples of the functional group conversion reaction that is further performed after the bond formation reaction include an oxidation reaction or a reduction reaction. Specific examples thereof include a reaction in which a sulfur atom is oxidized and converted to a sulfoxide group or a sulfone group. Other examples thereof include a reduction reaction in which a triple bond or a double bond among carbon-carbon bonds is reduced and converted to a double bond or a single bond. Two amino acids may be bonded at the main chains of the amino acids to form a closed ring structure by a peptide bond, or a covalent bond may be formed between two amino acids via, for example, a bond between side chains or between a side chain and the main chain of the two amino acids.

As used herein, the term "cyclic moiety" of the cyclic peptide means a ring-like portion formed by linkage of 4 or more amino acid residues.

In the present embodiment, the number of amino acid residues forming the cyclic moiety of the cyclic peptide is not particularly limited. It may be, for example, 5 or more, 6 or more, 7 or more, 8 or more, 9 or more, 10 or more, or 11 or more, and may be 15 or less, 14 or less, 13 or less, 12 or less, or 11 or less. The number of amino acid residues forming the cyclic moiety of the cyclic peptide in the present embodiment is not particularly limited, and may be, for example, 5 or more and 15 or less, 6 or more and 15 or less, 7 or more and 15 or less, 8 or more and 15 or less, 9 or more and 15 or less, 10 or more and 14 or less, 11 or more and 13 or less, 11 or more and 12 or less, or 11. The number of amino acid residues forming the cyclic moiety of the cyclic peptide in the present embodiment is not particularly limited, and is, for example, 5 or more and 15 or less, preferably 10 or more and 14 or less, more preferably 11 or more and 13 or less, and most preferably 11.

In the present embodiment, the cyclic moiety of the cyclic peptide is not particularly limited, and may be composed of, for example, a 28- to 55-, 28- to 49-, 31- to 46-, 34- to 43-, 34-to 40-, 34- to 37-, or 34-membered ring. The cyclic moiety of the cyclic peptide in the present embodiment is not particularly limited, and is composed of, for example, a 28- to 55-membered ring, preferably a 31- to 46-membered ring, more preferably 34- to 37-membered ring, and most preferably 34-membered ring.

The term "N-substituted amino acid" as used herein means an amino acid in which the amino group contained in the amino acid is substituted, that is, represented by -NHR (R represents alkyl, alkenyl, alkynyl, aryl, heteroaryl, aralkyl, or cycloalkyl optionally having a substituent, and one or two non-adjacent methylene groups in any of these groups is optionally substituted by an oxygen atom, a carbonyl group (-CO-), or a sulfonyl group (-SO₂-); and a carbon chain bonded to the N atom and the carbon atom at position α may form a ring, as in proline).

The N-substituted amino acid in the present embodiment is not particularly limited. It may be an N-alkyl amino acid, and may form a ring with a nitrogen atom forming the main chain and a carbon atom bonded to the nitrogen atom. The "N-substituted amino acid" in the present embodiment is preferably an N-C₁-C₆ alkyl amino acid, and may form a ring with a nitrogen atom forming the main chain and a carbon atom bonded to the nitrogen atom. The "N-substituted amino acid" in the present embodiment is more preferably an N-ethyl amino acid or an N-methyl amino acid, and most preferably an N-methyl amino acid.

The number of N-substituted amino acid residues contained in the peptide in the present embodiment is not particularly limited, and may be, for example, 1 or more, 2 or more, 3 or more, 4 or more, or 5 or more. The number of N-substituted amino acid residues contained in the peptide in the present embodiment is not particularly limited, and is, for example, 1 or more, preferably 3 or more, more preferably 4 or more, and most preferably 5 or more.

The term "N-unsubstituted amino acid" as used herein means an amino acid in which an amino group contained in the amino acid is not substituted, that is, represented by -NH₂. Preferred examples of the "N-unsubstituted amino acid" as used herein include an N-unsubstituted amino acid in which the amino group contained in the "main chain of an amino acid" is not substituted.

The number of N-unsubstituted amino acid residues contained in the peptide in the present embodiment is not particularly limited, and may be, for example, 1 or more, 2 or more, or 3 or more. The number of N-unsubstituted amino acid residues contained in the peptide in the present embodiment is not particularly limited, and is, for example, 1 or more, preferably 2 or more, more preferably 3 or more, and most preferably 3 or more.

As used herein, the "amino acid residue" forming a peptide is sometimes referred to simply as an "amino acid".

The term "side chain of an amino acid" as used herein means, in the case of an α-amino acid, an atomic group bonded to carbon (α-carbon) to which an amino group and a carboxyl group are bonded, other than the amino group and the carboxyl group. For example, the methyl group of Ala is a side chain of the amino acid. In the case of a β-amino acid, an atomic group bonded to the α-carbon and/or the β-carbon, other than the amino group bonded to the β-carbon and the carboxyl group bonded to the α-carbon, can be a side chain of the amino acid. Also, in the case of a γ-amino acid, an atomic group bonded to the α-carbon, the β-carbon, and/or the γ-carbon, other than the amino group bonded to the γ-carbon and the carboxyl group bonded to the α-carbon, can be a side chain of the amino acid.

The term "main chain of an amino acid" in the present specification means a chain portion formed by an amino group, α-carbon and a carboxyl group in the case of an α-amino acid, a chain portion formed by an amino group, β-carbon, α-carbon and a carboxyl group in the case of a β-amino acid, and a chain portion formed by an amino group, γ-carbon, β-carbon, α-carbon and a carboxyl group in the case of a γ-amino acid. Also, the term "α-amino acid skeleton" means a chain portion constituted by the amino group, the α-carbon, and the carboxyl group; the term "β-amino acid skeleton" means a chain portion constituted by the amino group, the β-carbon, the α-carbon, and the carboxyl group; and the term "γ-amino acid skeleton" means a chain portion constituted by the amino group, the γ-carbon, the β-carbon, the α-carbon, and the carboxyl group. As used herein, an amino acid having a "β-amino acid skeleton" as a whole or as a substructure may be referred to as an "amino acid having a β-amino acid skeleton". For example, L-aspartic acid has a chain portion constituted by the amino group, the β-carbon, the α-carbon, and the carboxyl group (β-amino acid skeleton), and thus falls under the category of the "amino acid having a β-amino acid skeleton".

The peptide in the present embodiment may contain at least one β-amino acid skeleton, and preferably contains at least one β-amino acid skeleton.

When the peptide in the present embodiment has a cyclic moiety, the peptide in the present embodiment may contain at least one β-amino acid skeleton in the cyclic moiety, and preferably contains at least one β-amino acid skeleton in the cyclic moiety.

The term "substituted hydroxyphenyl group" as used herein means a group in which at least one hydrogen atom of the aromatic ring of the hydroxyphenyl group is replaced with a substituent. The substituent is not particularly limited, and is freely selected from a hydrogen atom as well as, for example, a halogen, an alkyl group, an alkenyl group, an alkynyl group, an aryl group, a heteroaryl group, an aralkyl group, and a cycloalkyl group. One or two non-adjacent methylene groups in any of these groups may be substituted by an oxygen atom, a carbonyl group (-CO-), or a sulfonyl group (-SO₂-). Each of the side chains may have a substituent. The substituent is also not limited, and one or two or more substituents may be freely selected independently from any substituents including, for example, a halogen atom, an O atom, an S atom, an N atom, a B atom, a Si atom, or a P atom. Specifically, examples thereof include an optionally substituted alkyl group, alkenyl group, alkynyl group, aryl group, heteroaryl group, aralkyl group, and cycloalkyl group, and a halogen is preferred, with fluorine being particularly preferred. Examples of the substituted hydroxyphenyl group include, but are not intended to be limited to, a 3-fluoro-4-hydroxyphenyl group. The hydrogen atom of the aromatic ring as used herein does not include H of the hydroxy group (-OH) in the hydroxyphenyl group. For example, either the "substituted hydroxyphenyl group" or the "unsubstituted hydroxyphenyl group" as used herein does not include a methoxyphenyl group.

The term "unsubstituted hydroxyphenyl group" as used herein means an unsubstituted hydroxyphenyl group. The substituted hydroxyphenyl group and the unsubstituted hydroxyphenyl group may be collectively referred to as a "substituted or unsubstituted hydroxyphenyl group".

The phrase "does not have a substituted or unsubstituted XX group" as used herein means that either a substituted XX group or an unsubstituted XX group is not present.

The peptide in the present embodiment may have no indolyl group, may have no substituted or unsubstituted hydroxyphenyl group, and may have no indolyl group and substituted or unsubstituted hydroxyphenyl group. The peptide in the present embodiment preferably has no indolyl group, or has no substituted or unsubstituted hydroxyphenyl group, and more preferably has no indolyl group and substituted or unsubstituted hydroxyphenyl group.

The term "number of aromatic rings" (also referred to as an "aromatic ring count" (ARC)) as used herein refers to the number of aromatic rings contained in a peptide moiety other than a nucleic acid linkage moiety, a cyclic moiety or a side chain of a cyclic moiety of a cyclic peptide compound. For example, a phenol group is counted as 1, a bicyclic condensed ring such as an indole skeleton is counted as 2, and a tricyclic condensed ring such as anthracene is counted as 3.

The number of aromatic rings contained in the peptide in the present embodiment is not particularly limited, and may be, for example, 0 to 3 or 1 to 3. The number of aromatic rings contained in the peptide in the present embodiment is not particularly limited, and is, for example, 0 to 3, preferably 0 to 3, more preferably 1 to 3, and most preferably 1 to 3.

The term "pKa" as used herein refers to a measured value of pKa unless otherwise specified. The pKa value determined by ADMET Predictor described later is referred to as calculated pKa. The term "basic pKa" as used herein refers to a measured value of basic pKa unless otherwise specified. The basic pKa value determined by ADMET Predictor described later is referred to as basic calculated pKa.

The pKa and basic pKa can be measured by a conventional method. The measurement can be performed by, for example, the method described in Experimental Chemistry Lecture, vol. 5, "Thermal Measurements and Equilibrium", pp. 460 (edited by the Chemical Society of Japan, published by Maruzen Publishing Co., Ltd.). When the pKa value and the basic pKa value, to be determined, of a side chain of an amino acid are difficult to determine due to the influence of other functional groups, the measurement can be performed with the other functional groups protected by protecting groups or the like so that only the pKa and basic pKa of an intended functional group can be measured.

As used herein, the term "acidic side chain" means a side chain having a pKa of 10 or less, and the term "basic side chain" means a side chain having a basic pKa of 4 or more. As used herein, a side chain having a pKa of more than 10 and a side chain having a basic pKa of less than 4 are each defined as a neutral side chain.

As used herein, the calculated pKa and basic calculated pKa of a side chain of an amino acid, or a side chain of a cyclic moiety of a cyclic peptide compound can be determined using ADMET Predictor (Simulations Plus Inc., ver. 8.0). The calculated pKa and the basic calculated pKa are calculated using a partial structure obtained by extracting a side chain moiety at the E-position on the side chain (carbon directly bonded to the main chain). As an example, the case of Lys is shown below. The basic calculated pKa calculated using a partial structure including the β-position on the side chain (carbon directly bonded to the main chain) was 10.5. Calculation similarly performed for acids shows that the calculated pKa of the side chain carboxy group of Asp was 4.3, the calculated pKa of the side chain phenolic hydroxyl group of Tyr was 9.9, the calculated pKa of the side chain phenolic hydroxyl group of 3-fluorotyrosine (Tyr (3-F)) was 8.7, and the calculated pKa of tetrazole was 3.7. On the other hand, for bases, the basic calculated pKa of the side chain guanidino group of Arg was 12.7, the basic calculated pKa of the imidazolyl group of His was 7.6, and the basic calculated pKa of pyridine was 5.4.

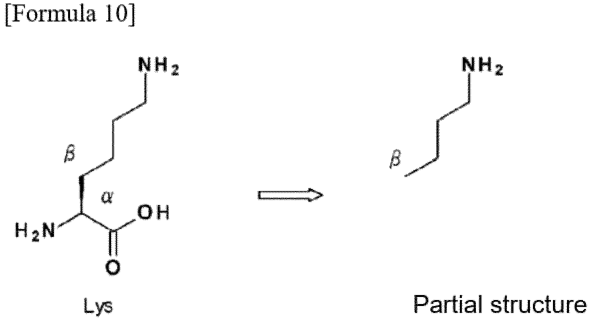

When the peptide in the present embodiment has an acidic side chain, the pKa of the acidic side chain is not particularly limited, and it may be, for example, 3.5 or more, 4.0 or more, 4.5 or more, or 5.0 or more, and may be 10 or less. When the peptide in the present embodiment has an acidic side chain, the pKa of the acidic side chain is not particularly limited, and may be, for example, 3.5 to 10, 4.0 to 10, 4.5 to 10, or 5.0 to 10. When the peptide in the present embodiment has an acidic side chain, the pKa of the acidic side chain is not particularly limited, and is, for example, 3.5 to 10, preferably 4.0 to 10, more preferably 4.5 to 10, and most preferably 5.0 to 10.

When the peptide in the present embodiment has a basic side chain, the basic pKa of the basic side chain is not particularly limited, and it may be, for example, 4.0 or more, and may be 10 or less, 9.0 or less, 8.5 or less, 8.0 or less, 7.5 or less, or 7.2 or less. When the peptide in the present embodiment has a basic side chain, the basic pKa of the basic side chain is not particularly limited, and may be, for example, 4.0 to 10, 4.0 to 9.0, 4.0 to 8.5, 4.0 to 8.0, 4.0 to 7.5, or 4.0 to 7.2. When the peptide in the present embodiment has a basic side chain, the basic pKa of the basic side chain is not particularly limited, and is, for example, 4.0 to 10, preferably 4.0 to 8.5, more preferably 4.0 to 7.5, and most preferably 4.0 to 7.2.

As used herein, the term "ClogP" means a distribution coefficient calculated by a computer. ClogP can be determined according to the principle described in Daylight Version 4.9 of Daylight Chemical Information Systems, Inc. (https://www.daylight.com/dayhtml/doc/clogp/). Examples of the method of calculating ClogP include calculating using Daylight Version 4.95 (release date: August 1, 2011, ClogP algorithm version 5.4, database version 28, https://www.daylight.com/dayhtml/doc/release_notes/index.html) of Daylight Chemical Information Systems, Inc.

In the present embodiment, the ClogP of the peptide is not particularly limited. It may be, for example, 4 or more, 5 or more, 6 or more, 7 or more, 8 or more, or 9 or more, and may be 25 or less, 24 or less, 23 or less, 22 or less, or 21 or less, or 20 or less. In the present embodiment, the ClogP of the peptide is not particularly limited, and may be, for example, 4 or more and 25 or less, 5 or more and 24 or less, 6 or more and 23 or less, 7 or more and 22 or less, 8 or more and 21 or less, or 9 or more and 20 or less. In the present embodiment, the ClogP of the peptide is not particularly limited, and is, for example, 4 or more and 25 or less, preferably 6 or more and 23 or less, more preferably 8 or more and 21 or less, and most preferably 9 or more and 20 or less.

In the present embodiment, the ClogP of the peptide is not particularly limited, and may be, for example, equal to or greater than a peptide (CP02) shown below.

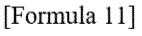

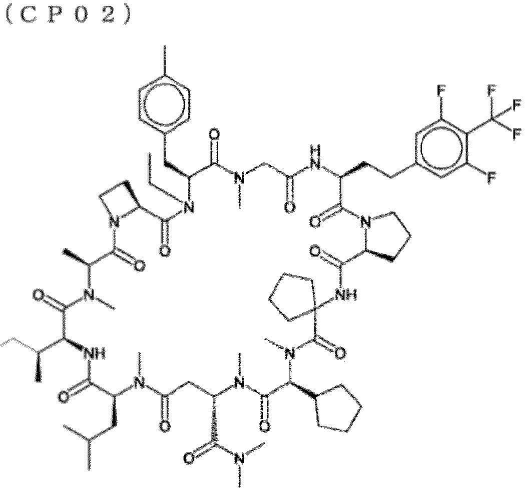

As used herein, the term "ClogP/number of amino acid residues" refers to a value calculated by dividing the ClogP of a peptide compound by the number of amino acid residues contained in the peptide compound. For example, when the ClogP of a peptide compound is 14.0 and the number of amino acid residues contained in the peptide compound is 7, the ClogP/number of amino acid residues of the peptide compound is calculated to be 2.0.

In the present embodiment, the ClogP/number of amino acid residues of the peptide is not particularly limited. It may be, for example, 1.0 or more, or 1.1 or more, and may be 1.8 or less, 1.7 or less, 1.6 or less, or 1.5 or less. In the present embodiment, the ClogP/number of amino acid residues of the peptide is not particularly limited, and may be, for example, 1.0 or more and 1.8 or less, 1.0 or more and 1.7 or less, 1.1 or more and 1.6 or less, or 1.1 or more and 1.5 or less. The ClogP/number of amino acid residues of the peptide is not particularly limited, and is, for example, 1.0 or more and 1.8 or less, preferably 1.0 or more and 1.7 or less, more preferably 1.1 or more and 1.6 or less, and most preferably 1.1 or more and 1.5 or less.

In the present embodiment, the peptide is not particularly limited. For example, the following peptides of (ECP1) to (ECP5) may be excluded.

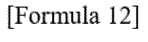

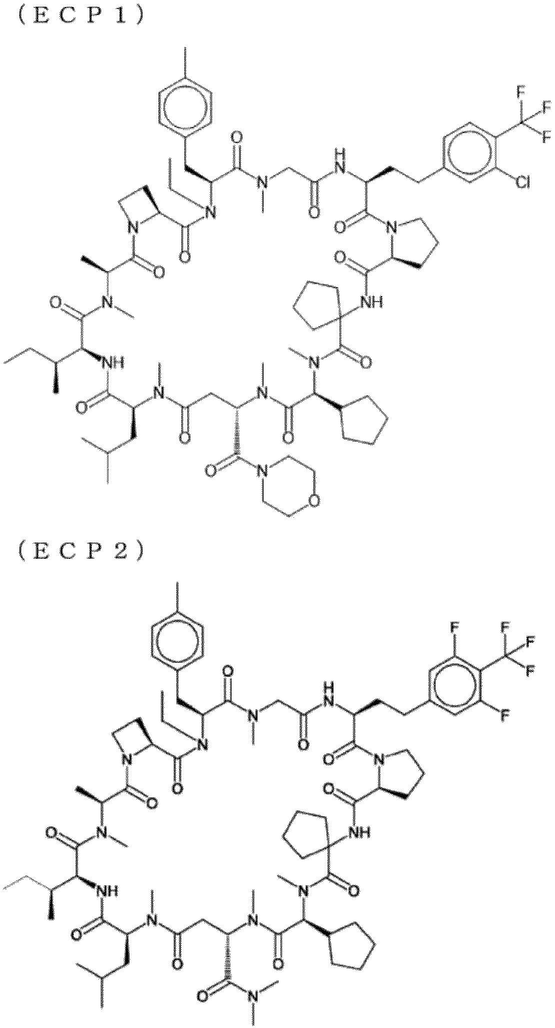

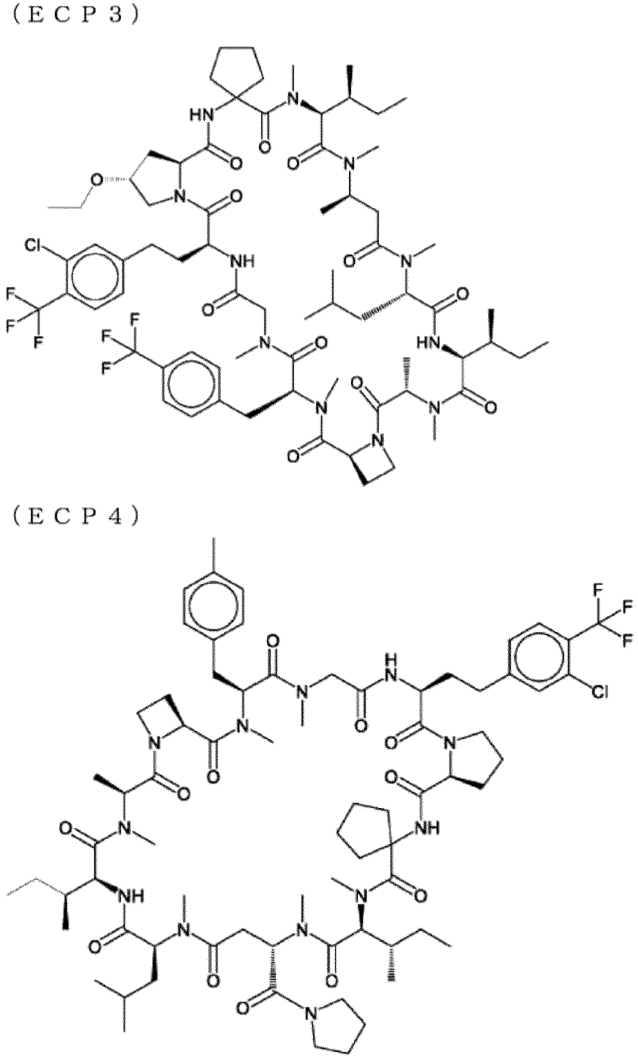

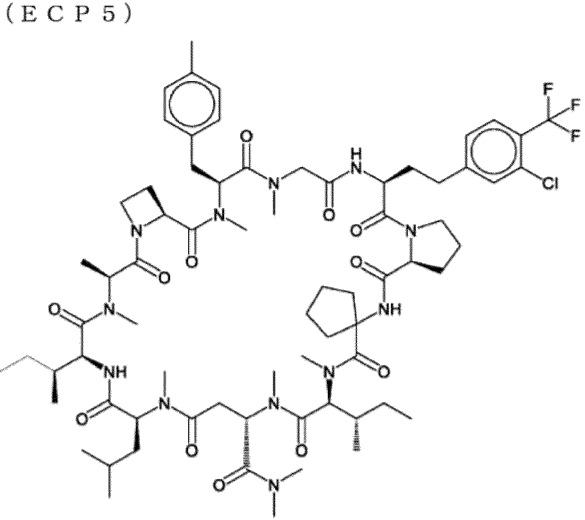

In the present embodiment, the peptide may be an amorphous substance. In the present embodiment, the peptide may be a crystalline substance.

### [Surfactant]

In the present embodiment, the surfactant is one or more selected from the group consisting of the following (I) to (III). In the present embodiment, the surfactant is preferably one or more selected from the group consisting of the following (I) and (II).
(I) An anionic surfactant;
(II) an amphoteric surfactant; and
(III) a nonionic surfactant.

In a preferred aspect of the present embodiment, the surfactant may be, for example, an anionic surfactant, and is preferably an alkyl sulfate, more preferably a lauryl sulfate, and most preferably sodium lauryl sulfate (also known as sodium dodecyl sulfate).

The anionic surfactant in the present embodiment is not particularly limited, and examples thereof include a fatty acid salt, a rosin acid salt, an alkyl sulfate, an alkyl polyoxyethylene sulfate, an alkyl naphthalene sulfate, a lignin sulfate, and an alkyl phosphate. They may be used alone, or used in combination of two or more thereof. The anionic surfactant in the present embodiment may be, for example, a fatty acid salt, a rosin acid salt, an alkyl sulfate, an alkyl polyoxyethylene sulfate, an alkyl naphthalene sulfate, a lignin sulfate, or an alkyl phosphate, and is preferably an alkyl sulfate, more preferably a lauryl sulfate, and most preferably sodium lauryl sulfate.

In another preferred aspect of the present embodiment, the surfactant may be, for example, an amphoteric surfactant, and is preferably acylcarnitine, more preferably lauroylcarnitine, and most preferably lauroyl-L-carnitine.

The amphoteric surfactant in the present embodiment is not particularly limited, and examples thereof include acylcarnitine, N-alkyl β-aminopropionic acid, N-alkyl sulfobetaine, and N-alkyl hydroxysulfobetaine. They may be used alone, or used in combination of two or more thereof. The amphoteric surfactant in the present embodiment is preferably acylcarnitine, N-alkyl β-aminopropionic acid, N-alkyl sulfobetaine or N-alkyl hydroxysulfobetaine, and more preferably acylcarnitine. The acylcarnitine is preferably lauroylcarnitine or carnitine palmitate, more preferably lauroylcarnitine, and most preferably lauroyl-L-carnitine.

The nonionic surfactant in the present embodiment is not particularly limited, and examples thereof include alkyl polyoxyethylene ether, alkyl aryl polyoxyethylene ether, a polyoxyethylene fatty acid ester, a polyoxyethylene glycerin fatty acid ester, a sorbitan fatty acid ester, a sucrose fatty acid ester, a polyglycerin fatty acid ester, a polyoxyethylene sorbitan fatty acid ester, poloxamer (poly(ethylene glycol)-block-poly(propylene glycol)-block-poly(ethylene glycol)), polyoxyethylene hydrogenated castor oil, and D-α-tocopherol polyethylene glycol 1000 succinate. They may be used alone, or used in combination of two or more thereof.

Among all the surfactants, anionic surfactants are preferred, lauryl sulfates are more preferred, and sodium lauryl sulfate is most preferred.

### [Polymer]

In the present embodiment, the polymer is selected from the group consisting of the following (IV) to (VIII).
(IV) hydroxypropyl methyl cellulose or a derivative thereof;
(V) a copolymer of a (meth)acrylic acid alkyl ester and at least one monomer selected from the group consisting of a (meth)acrylic acid, a (meth)acrylic acid alkyl ester, a (meth)acrylic acid alkylaminoalkyl ester and a (meth)acrylic acid ammonioalkyl ester;
(VI) copovidone;
(VII) a polyvinylcaprolactam-polyvinyl acetate-polyethylene glycol graft copolymer; and
(VIII) polyvinylpyrrolidone.

When the composition according to an aspect of the present embodiment contains a peptide as a solid dispersion that is a spray-dried product of a mixture of the peptide and a polymer, the polymer may be one or more selected from the group consisting of the above (IV) to (VII), and is preferably one or more selected from the group consisting of (IV), (V) and (VII), more preferably one or more selected from the group consisting of (IV) and (V), and most preferably (IV). By using these polymers, solubility of the peptide can be efficiently enhanced.

When the composition according to an aspect of the present embodiment contains a peptide as a solid dispersion of a mixture of the peptide and a polymer, the polymer may be one or more selected from the group consisting of the above (IV) and (VII), and is preferably one or more selected from the group consisting of (IV) and (VII), more preferably (IV), and most preferably (IV). By using these polymers, solubility of the peptide can be efficiently enhanced.

In an aspect of the present embodiment, when the peptide is a peptide (CP02) represented by the following formula: 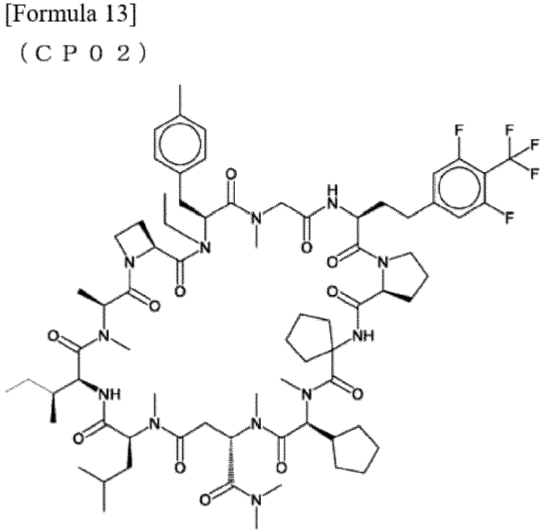 the polymer may be one or more polymers selected from the group consisting of the above (IV), (V) and (VII), and is preferably one or more selected from the group consisting of (IV) and (VII), more preferably one or more selected from the group consisting of (IV) and (VII), and preferably (IV). By using these polymers, solubility of CP02 can be efficiently enhanced.

The polymers according to the present embodiment may be used alone, or used in combination of two or more thereof.

In the present embodiment, the polymer is preferably an ionic polymer, and more preferably an acidic polymer.

As used herein, the term "ionic polymer" means a polymer which has a substantially ionic functional group, and is ionized by at least about 10% over at least a part of a physiologically relevant pH range of 1 to 8. Examples of the ionic polymer include an acidic polymer and a basic polymer. The ionic polymer is classified generally into an acidic polymer and a basic polymer according to a pH range in which the polymer is ionized, and the acidic polymer (or an enteric polymer) dissolves in a neutral or alkaline solution.

In the present embodiment, examples of the acidic polymer include cellulose acetate phthalate, cellulose acetate trimellitate, cellulose acetate succinate, methyl cellulose phthalate, hydroxymethyl cellulose ethyl phthalate, hydroxypropyl methyl cellulose phthalate (HPMCP), hydroxypropyl methyl cellulose acetate succinate (HPMCAS), hydroxypropyl methyl acetate maleate, hydroxypropyl methyl trimellitate, carboxymethyl ethyl cellulose, polyvinyl butyrate phthalate, polyvinyl alcohol acetate phthalate, a methacrylic acid/ethyl acrylate copolymer, a methacrylic acid/methyl methacrylate copolymer, a copolymer of acrylic acid and methacrylic acid, a copolymer of a (meth)acryric acid alkyl ester and (meth)acrylic acid, a copolymer of a (meth)acrylic acid alkyl ester and a (meth)acrylic acid alkylaminoalkyl ester, and a copolymer of a (meth)acrylic acid alkyl ester and a (meth)acrylic acid ammonioalkyl ester.

In the present embodiment, examples of the basic polymer include an aminoalkyl methacrylic acid copolymer E, and polyvinyl acetal diethyl aminoacetate.

In a preferred aspect of the present embodiment, the polymer may be hydroxypropyl methyl cellulose or a derivative thereof, and is preferably hydroxypropyl methyl cellulose or an ester thereof, more preferably one or more selected from the group consisting of hydroxypropyl methyl cellulose acetate succinate (HPMCAS), hydroxypropyl methyl cellulose phthalate (HPMCP), hydroxypropyl methyl acetate maleate and hydroxypropyl methyl trimellitate, and most preferably hydroxypropyl methyl cellulose acetate succinate (HPMCAS).

The hydroxypropyl methyl cellulose is also known as hypromellose. As used herein, the term "derivative of hydroxypropyl methyl cellulose" means a polymer in which the hydroxyl group of hydroxypropyl methyl cellulose is reacted (modified), and examples thereof include an ester, ether, carbamate and carbonate of hydroxypropyl methyl cellulose. In the derivative of hydroxypropyl methyl cellulose according to the present embodiment, the proportion of modified hydroxyl groups in hydroxyl groups of hydroxypropyl methyl cellulose is not particularly limited, and may be, for example, 10% or more, 200;ó or more, 30% or more, 40% or more, 50% or more, 60% or more, 70% or more, or 80% or more.

The hydroxypropyl methyl cellulose or a derivative thereof in the present embodiment is not particularly limited, and is preferably hydroxypropyl methyl cellulose or an ester thereof, more preferably one or more selected from the group consisting of hydroxypropyl methyl cellulose acetate succinate (HPMCAS), hydroxypropyl methyl cellulose phthalate (HPMCP), hydroxypropyl methyl acetate maleate and hydroxypropyl methyl trimellitate, and most preferably hydroxypropyl methyl cellulose acetate succinate (HPMCAS).

In an aspect of the present embodiment, the polymer may be, for example, a copolymer of a (meth)acrylic acid alkyl ester and at least one monomer selected from the group consisting of (meth)acrylic acid, a (meth)acrylic acid alkyl ester, a (meth)acrylic acid alkylaminoalkyl ester and a (meth)acrylic acid ammonioalkyl ester, and is preferably a copolymer of a (meth)acrylic acid alkyl ester and at least one monomer selected from the group consisting of (meth)acrylic acid, a (meth)acrylic acid alkyl ester and a (meth)acrylic acid alkylaminoalkyl ester, more preferably a copolymer of a (meth)acrylic acid alkyl ester and at least one monomer selected from the group consisting of (meth)acrylic acid and a (meth)acrylic acid alkylaminoalkyl ester, and most preferably a copolymer of a (meth)acrylic acid alkyl ester and (meth)acrylic acid. In this case, the copolymer of a (meth)acrylic acid alkyl ester and (meth)acrylic acid is preferably one or more selected from the group consisting of a copolymer of methyl methacrylate and methacrylic acid and a copolymer of ethyl acrylate and methacrylic acid, and more preferably a copolymer of methyl methacrylate and methacrylic acid. When a (meth)arylic acid alkyl ester and a (meth)acrylic acid alkyl ester are combined, the combination thereof is a combination of those having different types of alkyl esters, or a combination of a methacrylic acid alkyl ester and an acrylic acid alkyl ester.

As used herein, the term "(meth)acrylic acid" means "acrylic acid and/or methacrylic acid".

The (meth)acrylic acid in the present embodiment is not particularly limited, and is preferably methacrylic acid.

As used herein, the term "(meth)acrylic acid alkyl ester" means an "acrylic acid alkyl ester and/or methacrylic acid alkyl ester". The (meth)acrylic acid ester in the present embodiment is not particularly limited. It may be, for example, a (meth)acrylic acid Ci-Cs alkyl ester, and is preferably a (meth)acrylic acid methyl ester or a (meth)acrylic acid ethyl ester, more preferably methyl methacrylate or ethyl acrylate, and most preferably methyl methacrylate.

The term "(meth)acrylic acid alkylaminoalkyl ester" as used herein means an "acrylic acid alkylaminoalkyl ester" or "methacrylic acid alkylaminoalkyl ester". The (meth)acrylic acid alkylaminoalkyl ester in the present embodiment is not particularly limited, and is preferably 2-(dimethylamino)ethyl (meth)acrylate, and more preferably 2-(dimethylamino)ethyl methacrylate.

The term "(meth)acrylic acid ammonioalkyl ester" as used herein means an "acrylic acid ammonioalkyl ester" or methacrylic acid ammonioalkyl ester". The (meth)acrylic acid ammonioalkyl ester in the present embodiment is not particularly limited. It may be, for example, trialkylammonium alkyl (meth)actylic acid chloride ([(meth)acryloyloxyalkyl]trialkylammonium chloride), and is preferably trimethylammonium ethyl methacrylic acid chloride ([2-(methacryloyloxy)ethyl]trimethylammonium chloride).

In the copolymer of the (meth)acrylic acid alkyl ester according to the present embodiment and at least one monomer selected from the group consisting of (meth)acrylic acid, a (meth)acrylic acid alkyl ester, a (meth)acrylic acid alkylaminoalkyl ester and a (meth)acrylic acid ammonioalkyl ester, the ratio of a unit derived from the (meth)acrylic acid alkyl ester and a unit derived from at least one monomer selected from the group consisting of (meth)acrylic acid, a (meth)acrylic acid alkyl ester, a (meth)acrylic acid alkylaminoalkyl ester and a (meth)acrylic acid ammonioalkyl ester is not particularly limited. It may be, for example, 10 : 90 to 90 : 10, 20 : 80 to 80 : 20, 30 : 70 to 70 : 30, 40 : 60 to 60 : 40 or 45 : 55 to 55 : 45, and is preferably 40 : 60 to 60 : 40, on a molar basis.

The number average molecular weight of the copolymer of the (meth)acrylic acid alkyl ester according to the present embodiment and at least one monomer selected from the group consisting of (meth)acrylic acid, a (meth)acrylic acid alkyl ester, a (meth)acrylic acid alkylaminoalkyl ester and a (meth)acrylic acid ammonioalkyl ester is not particularly limited, and may be, for example, 10,000 g/mol or more and 10,000,000 g/mol or less, 30,000 g/mol or more and 3,000,000 g/mol or less, or 100,000 g/mol or more and 1,000,000 g/mol or less.

The copovidone (also known as copolyvidone) is a copolymer of 1-vinyl-2-pyrrolidone and vinyl acetate, and the content ratio thereof is about 3 : 2 on a weight basis.

The polyvinylcaprolactam-polyvinyl acetate-polyethylene glycol graft copolymer is a polymer of graft copolymers composed of polyvinylcaprolactam, polyvinyl acetate and polyethylene glycol, which is a support polymer for a solid dispersion. The content ratio of polyvinylcaprolactam, polyvinyl acetate and polyethylene glycol in the polyvinylcaprolactam-polyvinyl acetate-polyethylene glycol graft copolymer according to the present embodiment is not particularly limited, and may be, for example, 13 : 57 : 30 on a weight basis. Examples of the polyvinylcaprolactam-polyvinyl acetate-polyethylene glycol graft copolymer include Soluplus (registered trademark, BASF SE).

The number average molecular weight in the polyvinylcaprolactam-polyvinyl acetate-polyethylene glycol graft copolymer according to the present embodiment is not particularly limited, and may be, for example, 10,000 g/mol or more and 10,000,000 g/mol or less, 30,000 g/mol or more and 3,000,000 g/mol or less, 10,000 g/mol or more and 1,000,000 g/mol or less, or 30,000 g/mol or more and 300,000 g/mol or less.

The polyvinylpyrrolidone (also known as popidone, povidone or polyvidone) is a polymer of N-vinyl-2-pyrrolidone.

Among all these polymers, hydroxypropyl methyl cellulose or a derivative thereof is preferred, one or more selected from the group consisting of hydroxypropyl methyl cellulose acetate succinate (HPMCAS), hydroxypropyl methyl cellulose phthalate (HPMCP), hydroxypropyl methyl acetate maleate and hydroxypropyl methyl trimellitate are more preferred, and hydroxypropyl methyl cellulose acetate succinate (HPMCAS) is most preferred.

### [Combination of surfactant and polymer]

In a preferred aspect of the present embodiment, the surfactant may be an anionic surfactant, and the polymer may be hydroxypropyl methyl cellulose or a derivative thereof.

In a preferred aspect of the present embodiment, the surfactant may be sodium lauryl sulfate, and the polymer may be hydroxypropyl methyl cellulose acetate succinate (HPMCAS).

In a preferred aspect of the present embodiment, the surfactant may be an anionic surfactant, and the polymer may be a copolymer of a (meth)acrylic acid alkyl ester and at least one monomer selected from the group consisting of (meth)acrylic acid, a (meth)acrylic acid alkyl ester, a (meth)acrylic acid alkylaminoalkyl ester and a (meth)acrylic acid ammonioalkyl ester.

In a preferred aspect of the present embodiment, the surfactant may be sodium lauryl sulfate, and the polymer may be a copolymer of methyl methacrylate and methacrylic acid.

In a preferred aspect of the present embodiment, the surfactant may be an amphoteric surfactant, and the polymer may be hydroxypropyl methyl cellulose or a derivative thereof.

In a preferred aspect of the present embodiment, the surfactant may be lauroyl-L-carnitine, and the polymer may be hydroxypropyl methyl cellulose acetate succinate (HPMCAS).

In a preferred aspect of the present embodiment, the surfactant may be an amphoteric surfactant, and the polymer may be a copolymer of a (meth)acrylic acid alkyl ester and at least one monomer selected from the group consisting of (meth)acrylic acid, a (meth)acrylic acid alkyl ester, a (meth)acrylic acid alkylaminoalkyl ester and a (meth)acrylic acid ammonioalkyl ester.

In a preferred aspect of the present embodiment, the surfactant may be lauroyl-L-carnitine, and the polymer may be a copolymer of methyl methacrylate and methacrylic acid.

In a preferred aspect of the present embodiment, the surfactant may be a nonionic surfactant, and the polymer may be hydroxypropyl methyl cellulose or a derivative thereof.

In a preferred aspect of the present embodiment, the surfactant may be a sucrose fatty acid ester, and the polymer may be hydroxypropyl methyl cellulose acetate succinate (HPMCAS).

In a preferred aspect of the present embodiment, the surfactant may be polyoxyethylene hydrogenated castor oil, and the polymer may be hydroxypropyl methyl cellulose acetate succinate (HPMCAS).

In a preferred aspect of the present embodiment, the surfactant may be D-α-tocopherol polyethylene glycol 1000 succinate, and the polymer may be hydroxypropyl methyl cellulose acetate succinate (HPMCAS).

In a preferred aspect of the present embodiment, the surfactant may be an anionic surfactant, and the polymer may be a polyvinylcaprolactam-polyvinyl acetate-polyethylene glycol graft copolymer.

In a preferred aspect of the present embodiment, the surfactant may be sodium lauryl sulfate, and the polymer may be a polyvinylcaprolactam-polyvinyl acetate-polyethylene glycol graft copolymer.

### [Composition]

In the composition according to the present embodiment, the ratio of the contents of the peptide and the surfactant is not particularly limited, and for example, the content of the surfactant to the content of the peptide on a weight basis is 0.1 or more and 40 or less, preferably 0.1 or more and 15.0 or less, more preferably 0.3 or more and 6.0 or less, and most preferably 0.5 or more and 3.0 or less.

In the composition according to the present embodiment, the ratio of the content of the peptide and the polymer is not particularly limited, and for example, the content of the polymer to the content of the peptide on a weight basis is 0.01 or more and 40 or less, preferably 0.01 or more and 15.0 or less, more preferably 0.03 or more and 6.0 or less, and most preferably 0.1 or more and 3.0 or less.

When the composition according to the present embodiment is a pharmaceutical composition, the pharmaceutical composition may contain pharmaceutically acceptable other components to the extent that the effect according to the present invention is not impaired. Examples of the other components include a stabilizer, a preservative, an antioxidant, a disintegrant, an excipient, a binder, and a fluidizer or lubricant. Examples of the stabilizer include phosphatidic acid, ascorbic acid, glycerin, and cetanol. Examples of the preservative include ethyl paraoxybenzoate and propyl paraoxybenzoate. Examples of the antioxidant include butylated hydroxytoluene, butylated hydroxyanisole, propyl gallate, and gallic acid propyl ester. Examples of the disintegrant include calcium carmellose, sodium croscarmellose, crospopidone, and low-substituted hydroxypropyl cellulose. Examples of the excipient include starches such as corn starch, lactose, glucose, and D-mannitol. Examples of the binder include sucrose, gelatin, gum arabic powder, and methylcellulose. Examples of the fluidizer or lubricant include light anhydrous silicic acid, aqueous silicic acid dioxide, magnesium stearate, and talc.

When the composition according to the present embodiment is a pharmaceutical composition, the shape of the pharmaceutical composition is not particularly limited, and is typically a solid. The pharmaceutical composition is molded into, for example, a powder, subtle granules, granules, a tablet, a coated tablet, a capsule and the like, and used.

When the composition according to the present embodiment is a pharmaceutical composition, the pharmaceutical composition may be orally administered, or may be parenterally administered. The pharmaceutical composition, which has enhanced solubility of the peptide, thus is preferably orally administered.

When the composition according to the present embodiment is a pharmaceutical composition, the subject to which the pharmaceutical composition is administered is not particularly limited, and may be a human or a non-human animal. Examples of the non-human animal include a dog, a monkey, a mini-pig, a rabbit, a rat, and a mouse.

When the composition according to the present embodiment is a pharmaceutical composition, the dosage of the pharmaceutical composition is not particularly limited, and it may be administered such that the dose of the peptide to be administered per body weight (kg) of the subject is 0.1 mg/kg or more and 1000 mg/kg or less, for example. The dose of the peptide to be administered may be, for example, 1 mg/kg or more and 500 mg/kg or less, 1 mg/kg or more and 100 mg/kg or less, 1 mg/kg or more and 50 mg/kg or less, 3 mg/kg or more and 30 mg/kg or less, 10 mg/kg or more and 30 mg/kg or less, 0.1 mg/kg or more and 10 mg/kg or less, 1 mg/kg or more and 5 mg/kg or less, 10 mg/kg or more and 100 mg/kg or less, 15 mg/kg or more and 50 mg/kg or less, 20 mg/kg or more and 40 mg/kg or less, 25 mg/kg or more and 35 mg/kg or less, 3 mg/kg, or 30 mg/kg.

The composition according to the present embodiments can be produced by being molded into an arbitrary dosage form by a method that is commonly used.

### [Evaluation and Use of Composition]

The solubility of the peptide contained in the composition according to the present embodiment can be evaluated by, for example, measuring the amount of dissolution of the peptide after adding the composition to a liquid in which the composition is to be dissolved, and incubating the mixture. The solution in which the composition is to be dissolved is not particularly limited, and may be, for example, digestive juice or an artificial solution prepared in imitation of digestive juice (artificial digestive juice) when the composition is a pharmaceutical composition in an oral dosage form. The artificial digestive juice may be, for example, artificial intestinal juice and/or artificial gastric juice. Examples of the artificial intestinal juice include FaSSIF Series (fasted state intestine, BIORELEVANT.COM LTD), FeSSIF Series (fed state intestine, BIORELEVANT.COM LTD), Fasscof Series (fasted state bowel, BIORELEVANT.COM LTD), and Fesscof Series (fed state bowel, BIORELEVANT.COM LTD), and examples of the artificial gastric juice include FaSSGF Series (fasted state stomach, BIORELEVANT.COM LTD). The amount of dissolution of the peptide can be measured by a method commonly used by those skilled in the art, and may be calculated by, for example, an internal standard method using an ultra-high-performance liquid chromatography method with a photodiode array detector.

As an example of specific evaluation of the solubility of the peptide for the composition according to the present embodiment, for example, the solubility of the peptide may be calculated by an internal standard method using an ultra-high-performance liquid chromatography method with a photodiode array detector after the composition or a control composition is added to FaSSIF with a pH of 6.5 at 37°C, the mixture is incubated, and the undissolved peptide is removed by filtration. For example, when the composition is a composition composed of a peptide, a surfactant, a polymer and DMSO, the control composition may be, for example, a composition composed of a peptide, a polymer and DMSO, or a composition composed of a peptide, a surfactant and DMSO. Here, it can be evaluated that a higher ratio of the value of the solubility of the peptide when the composition composed of the peptide, the surfactant, the polymer and DMSO is dissolved in FaSSIF with a pH of 6.5 at 37°C to the total value of the solubility of the peptide when the composition composed of the peptide, the polymer and DMSO is dissolved in FaSSIF with a pH of 6.5 at 37°C and the solubility of the peptide when the composition composed of the peptide, the surfactant and DMSO is dissolved in FaSSIF with a pH of 6.5 at 37°C leads to an enhanced solubility. For example, it may be evaluated that the solubility is enhanced when the ratio is more than 1.0, more than 1.1, more than 1.2, more than 1.3, more than 1.4, more than 1.5, more than 1.6, more than 1.7, more than 1.8, more than 1.9, more than 2.0, more than 2.1, more than 2.2 or more than 2.3.

The composition according to the present embodiment, which contains a peptide, a surfactant and a polymer, thus has enhanced solubility of the peptide in body fluid. In the composition according to present embodiment, the ratio of the value of the solubility of the peptide when the composition composed of the peptide, the surfactant, the polymer and DMSO is dissolved in FaSSIF with a pH of 6.5 at 37°C to the total value of the solubility of the peptide when the composition composed of the peptide, the polymer and DMSO is dissolved in FaSSIF with a pH of 6.5 at 37°C and the solubility of the peptide when the composition composed of the peptide, the surfactant and DMSO is dissolved in FaSSIF with a pH of 6.5 at 37°C is preferably more than 1.0, more preferably more than 1.3, and still more preferably more than 1.5.

The composition according to the present embodiment can be used as a pharmaceutical product containing a peptide as an active ingredient.

The composition according to the present embodiment may contain a peptide and a polymer as a solid dispersion. In other words, in the composition according to the present embodiment, the peptide and the polymer may form a solid dispersion.

The composition according to the present embodiment may contain a peptide and a polymer as a solid dispersion that is a spray-dried product of a mixture containing the peptide and the polymer. In other words, in the composition according to the present embodiment, the peptide and the polymer may form a solid dispersion obtained by spray drying. The peptide and the polymer in the composition according to the present embodiment may be derived from a solid dispersion containing the peptide and the polymer. In other words, the peptide and the polymer in the composition according to the present embodiment may be supplied from a solid dispersion containing the peptide and the polymer. Further, in other words, the peptide and the polymer in the composition according to the present embodiment may be contained in a solid dispersion containing the peptide and the polymer, and released from the solid dispersion in the composition.

In the solid dispersion according to the present embodiment, a substance to be dispersed is preferably dispersed with uniformity in the polymer. In the solid dispersion according to the present embodiment, the substance to be dispersed is preferably dispersed in very small units, and more preferably dispersed at a molecular level, in the polymer.

The solid dispersion according to the present embodiment refers to a semi-solid or solid substance in which a plurality of types of intended substances (for example, the peptide and polymer) exist in a state of being dispersed together. From the viewpoint of improving solubility, the plurality of types of substances are typically dispersed with uniformity, preferably dispersed with uniformity in very small units, more preferably dispersed with uniformity in very small units, and most preferably dispersed with uniformity at a molecular level, in a solid dispersion.

The method for forming a solid dispersion is not particularly limited, and examples thereof include a method in which a certain amount of the solvent or all the solvent is removed from a solution of the plurality of types of substances to form the solid dispersion, or a method in which a certain amount of a solvent is added to and simultaneously mixed with a plurality of types of substances that are solid. Specific examples thereof include a spray-drying method, a freeze-drying method, a precipitation method, a melt-extruding method, and a mixing/crushing method. A spray-drying method or a freeze-drying method is preferred, a spray-drying method or a freeze-drying method is more preferred, and a spray-drying method is most preferred. By using a spray-drying method, substances in the solid dispersion can be brought into a more uniformly dispersed state, and solubility of a substance contained in the solid dispersion (for example, a peptide) can be further improved. The solvent is only required to be capable of dissolving the plurality of types of substances, and examples thereof include water, alcohol (for example, methanol, ethanol, n-propanol, iso-propanol and butanol), ketone (for example, acetone, methyl ethyl ketone and methyl iso-butyl ketone), an ester (for example, ethyl acetate and propyl acetate), acetonitrile, methylene chloride, toluene, 1,1,1-trichloroethane, and tetrahydrofuran. The solvents may be used alone, or used as a mixed solvent obtained by mixing two or more thereof.

### [Other Embodiments]

Another embodiment of the present invention is a composition which contains a peptide and one or more polymers selected from the group consisting of the following (IV) to (VIII), and is intended to be used in combination with one or more surfactants selected from the group consisting of the following (I) to (III). An aspect of the present embodiment is a composition that is a solid dispersion, which contains a spray-dried product of a mixture containing a peptide and one or more polymers selected from the group consisting of the following (IV) to (VIII), and is intended to be used in combination with one or more surfactants selected from the group consisting of the following (I) to (III). An aspect of the present embodiment is a composition containing a solid dispersion, which contains a peptide and one or more polymers selected from the group consisting of the following (IV) and (VII), and is intended to be used in combination with one or more surfactants selected from the group consisting of the following (I) to (III). An aspect of the present embodiment is a composition which contains a peptide (CP02) represented by the following formula: 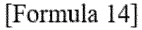 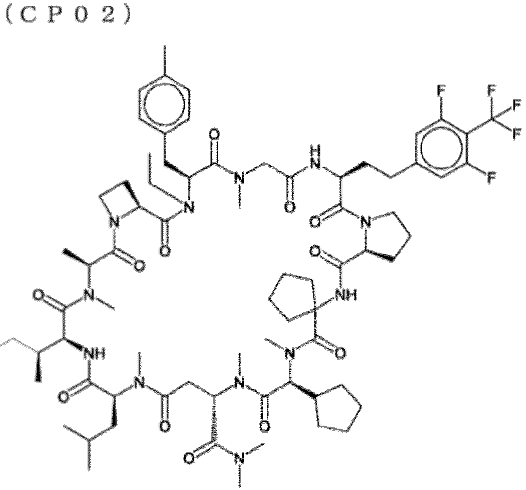 and one or more polymers selected from the group consisting of the following (IV), (V) and (VII), and is intended to be used in combination with one or more surfactants selected from the group consisting of the following (I) to (III). In these cases, as the composition for use in combination with the surfactant, one described above can be used except that a surfactant is not contained, and as the surfactant that is used in combination, one described above can be used. The composition according to the present embodiment may be a solid dispersion. The composition according to the present embodiment may contain a peptide and a polymer as a spray-dried product of a mixture containing the peptide and the polymer. The composition according to the present embodiment is preferably a pharmaceutical composition.
(I) An anionic surfactant;
(II) an amphoteric surfactant;
(III) a nonionic surfactant;
(IV) hydroxypropyl methyl cellulose or a derivative thereof;
(V) a copolymer of a (meth)acrylic acid alkyl ester and at least one monomer selected from the group consisting of a (meth)acrylic acid, a (meth)acrylic acid alkyl ester, a (meth)acrylic acid alkylaminoalkyl ester and a (meth)acrylic acid ammonioalkyl ester;
(VI) copovidone;
(VII) a polyvinylcaprolactam-polyvinyl acetate-polyethylene glycol graft copolymer; and
(VIII) polyvinylpyrrolidone.

Another embodiment of the present invention is a composition which contains one or more surfactants selected from the group consisting of the following (I) to (III), and is intended to be used in combination with a peptide and one or more polymers selected from the group consisting of the following (IV) to (VIII). In this case, as the peptide, the surfactant, the polymer and the solid dispersion, those described above can be used. The composition according to the present embodiment is preferably a pharmaceutical composition.
(I) An anionic surfactant;
(II) an amphoteric surfactant;
(III) a nonionic surfactant;
(IV) hydroxypropyl methyl cellulose or a derivative thereof;
(V) a copolymer of a (meth)acrylic acid alkyl ester and at least one monomer selected from the group consisting of a (meth)acrylic acid, a (meth)acrylic acid alkyl ester, a (meth)acrylic acid alkylamino alkyl ester and a (meth)acrylic acid ammonioalkyl ester;
(VI) copovidone;
(VII) a polyvinylcaprolactam-polyvinyl acetate-polyethylene glycol graft copolymer; and
(VIII) polyvinylpyrrolidone.

Another embodiment of the present invention is a method for improving solubility of a peptide by combining a peptide, one or more surfactants selected from the group consisting of the following (I) to (III), and one or more polymers selected from the group consisting of the following (IV) to (VIII). An aspect of the present embodiment is a method for improving solubility of a peptide by combining a peptide, a solid dispersion that is a spray-dried product of a mixture containing a peptide and one or more polymers selected from the group consisting of the following (IV) to (VIII), and one or more surfactants selected from the group consisting of the following (I) to (III). An aspect of the present embodiment is a method for improving solubility of a peptide by combining a solid dispersion containing a peptide and one or more polymers selected from the group consisting of the following (IV) and (VII) with one or more surfactants selected from the group consisting of the following (I) to (III). An aspect of the present embodiment is a method for improving solubility of a peptide by combining a peptide (CP02) represented by the following formula: 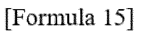 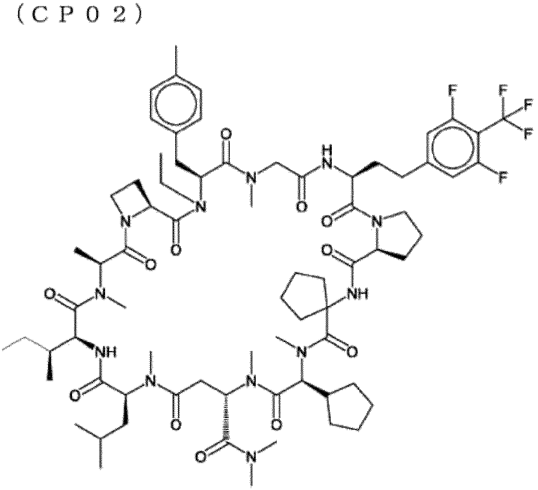 one or more surfactants selected from the group consisting of the following (I) to (III), and one or more polymers selected from the group consisting of the following (IV), (V) and (VII). In these cases, as the peptide, the surfactant, the polymer and the solid dispersion, those described above can be used.
(I) An anionic surfactant;
(II) an amphoteric surfactant;
(III) a nonionic surfactant;
(IV) hydroxypropyl methyl cellulose or a derivative thereof; and
(V) a copolymer of a (meth)acrylic acid alkyl ester and at least one monomer selected from the group consisting of (meth)acrylic acid, a (meth)acrylic acid alkyl ester, a (meth)acrylic acid alkylaminoalkyl ester and a (meth)acrylic acid ammonioalkyl ester; and
(VI) copovidone;
(VII) a polyvinylcaprolactam-polyvinyl acetate-polyethylene glycol graft copolymer; and
(VIII) polyvinylpyrrolidone.

Another embodiment of the present invention is a method for producing a composition, comprising the step of mixing a peptide, one or more surfactants selected from the group consisting of the following (I) to (III), and one or more polymers selected from the group consisting of the following (IV) to (VIII) to obtain the composition (mixing step). An aspect of the present embodiment is a method for producing a composition, comprising the step of combining a solid dispersion that is a spray-dried product of a mixture containing a peptide and one or more polymers selected from the group consisting of the following (IV) to (VIII) with one or more surfactants selected from the group consisting of the following (I) to (III) to obtain the composition (combination step). An aspect of the present embodiment is a method for producing a composition, comprising the step of combining a solid dispersion containing a peptide and one or more polymers selected from the group consisting of the following (IV) and (VII) with one or more surfactants selected from the group consisting of the following (I) to (III) to obtain the composition (combination step). An aspect of the present embodiment is a method for producing a composition, comprising the step of combining a peptide (CP02) represented by the following formula: 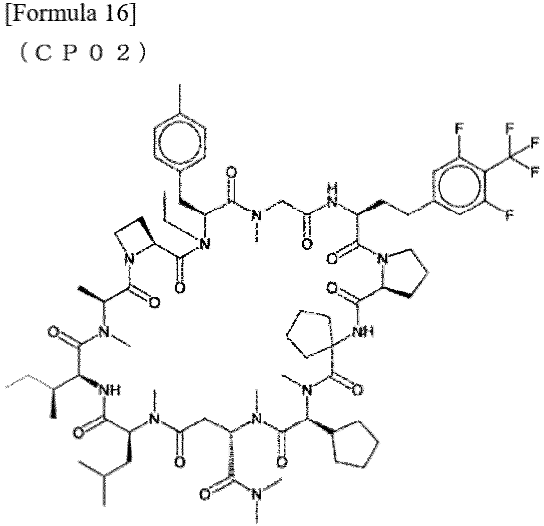 one or more surfactants selected from the group consisting of the following (I) to (III), and one or more polymers selected from the group consisting of the following (IV), (V) and (VII) to obtain the composition (mixing step). In these cases, as the peptide, the surfactant, the polymer and the solid dispersion, those described above can be used, and the composition produced by the production method according to the present embodiment may be a composition according to an embodiment of the present invention. In these cases, the combination of the peptide, the surfactant and the polymer includes mixing a peptide and a polymer, a solid dispersion that is a spray-dried product of a mixture containing a peptide and a polymer, or a solid dispersion containing a peptide and a polymer and a surfactant, molding them as a tablet, packing them together into a capsule, dissolving them in a solvent, and others such as making them coexist by some means.
(I) An anionic surfactant;
(II) an amphoteric surfactant;
(III) a nonionic surfactant;
(IV) hydroxypropyl methyl cellulose or a derivative thereof;
(V) a copolymer of a (meth)acrylic acid alkyl ester and at least one monomer selected from the group consisting of a (meth)acrylic acid, a (meth)acrylic acid alkyl ester, a (meth)acrylic acid alkylaminoalkyl ester and a (meth)acrylic acid ammonioalkyl ester; and
(VII) a polyvinylcaprolactam-polyvinyl acetate-polyethylene glycol graft copolymer.

The combination step may be carried out by a method commonly used by those skilled in the art, and may be carried out by, for example, a method in which a peptide and a polymer are added to a surfactant, or vice versa. In the combination step, in addition to the surfactant, the peptide and the polymer, other components that may be contained in the composition may be further combined. Examples of the other components include the above-described stabilizer, preservative, antioxidant, disintegrant, excipient, binder, fluidizer and lubricant.

In an aspect, the step of forming a solid dispersion containing a peptide and a polymer (solid dispersion forming step) may be further provided before the combination step. Examples for forming a solid dispersion in the solid dispersion forming step include the above-described method.

### [Examples]

The contents of the present invention will be further described by the following Examples, but the present invention is not limited to their contents. Except those as specifically described, starting substances, starting raw materials, solvents, and reagents were obtained from commercial suppliers, or synthesized using known methods. A hypromellose acetic acid ester succinate ester (hydroxypropyl methyl cellulose acetate succinate, HPMCAS, hydroxypropyl methyl cellulose or one of derivatives thereof) herein was obtained from Shin-Etsu Chemical Co., Ltd. (Shin-Etsu AQOAT LF) and used. Eudragit L100 which is a copolymer of methyl methacrylate and methacrylic acid was obtained from Evonik Industries AG. As sodium lauryl sulfate (SLS) which is an anionic surfactant, a product from BASF SE, or sodium dodecyl sulfate manufactured by FUJIFILM Wako Pure Chemical Corporation was used. Lauroyl-L-carnitine (LC) which is an amphoteric surfactant was obtained in the form of a hydrochloride from Sinochem Japan Co., Ltd. Polyvinylpyrrolidone (PVP K30), copovidone (Kollidon VA64) and a polyvinylcaprolactam-polyvinyl acetate-polyethylene glycol graft copolymer (Soluplus) were obtained from BASF SE and used. As a sucrose fatty acid ester (DK ester) which is a nonionic surfactant was obtained, DK ester SS was obtained from DKS Co. Ltd. and used. Polyoxyethylene hydrogenated castor oil 60 (HCO60) which is a nonionic surfactant was obtained from Nikko Chemicals Co., Ltd. and used. D-α-Tocopherol polyethylene glycol 1000 succinate (Vitamin E TPGS) which is a nonionic surfactant was obtained from Sigma-Aldrich Co. LLC and used. As FaSSIF which is human fasted state simulated intestinal fluid, SIF powder obtained from BIORELEVANT.COM LTD was dissolved at 3 mmol/L in terms of taurocholic acid and 0.75 mmol/L in terms of lecithin in a phosphate buffer solution containing sodium chloride, and the resulting solution was adjusted to a pH of 6.5, and used.

### [Production Example 1] Production of cyclic peptide and crystal thereof

Table 1 below shows cyclic peptides CP01 to CP04 used in the present Example. These compounds were synthesized by the method described in International Publication No. WO 2021/090855.

As the C-type crystal of CP02 described herein, one equivalent to the C-type crystal (hydrate crystal) of the peptide described in International Publication No. WO 2022/234864 was produced and used.

**[Table 1]**

| Name | Structure |
|---|---|
| C P 0 1 | 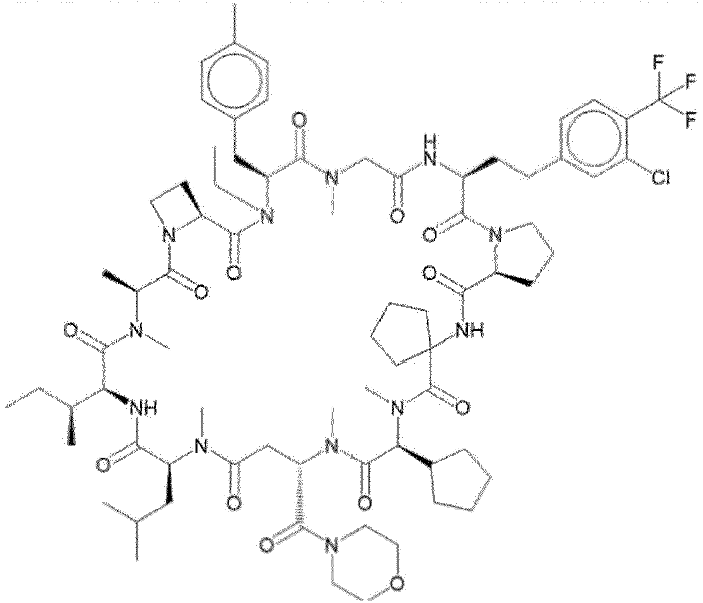 |
| C P 0 2 | 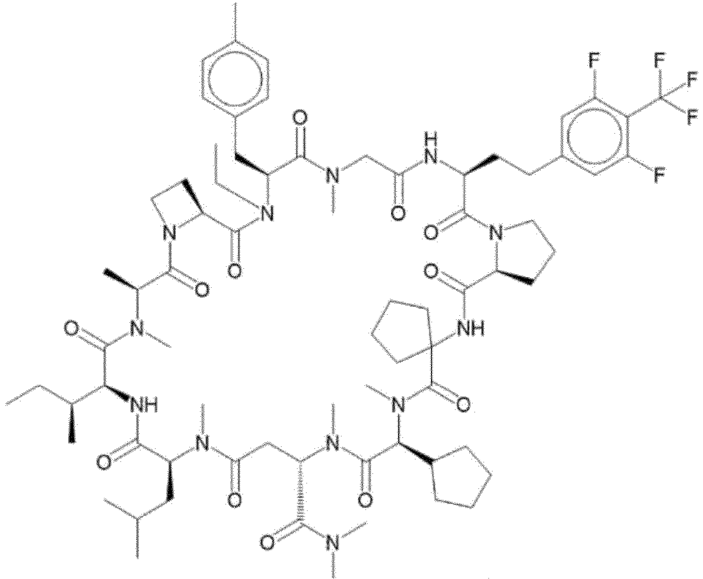 |
| C P 0 3 | 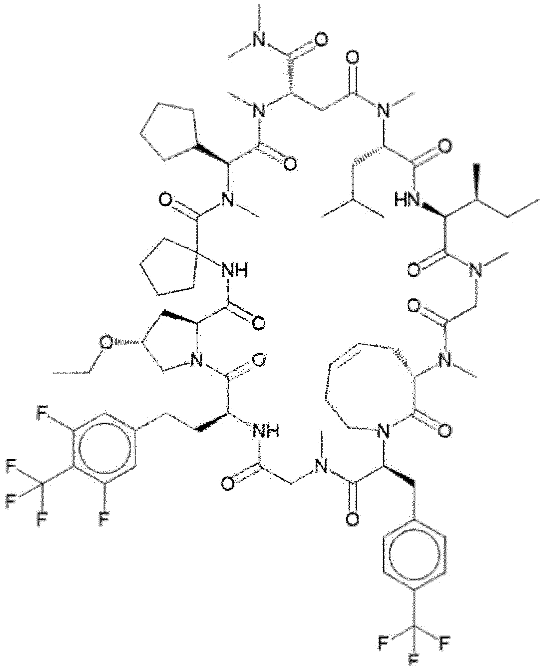 |
| C P 0 4 | 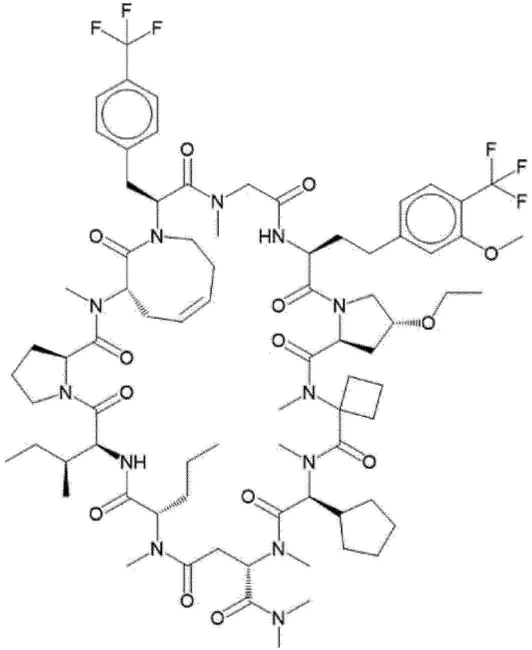 |

CP01: (3S,9S,12S,17S,20S,23S,27S,30S,36S)-3-[2-[3-chloro-4-(trifluoromethyl)phenyljethyl]-30-cyclopentyl-10-ethyl-23-isobutyl-7,17,18,24,28,31-hexamethyl-20-[(1S)-1-methylpropyl]-27-(morpholine-4-carbonyl)-9-(p-trylmethyl)spiro[ 1,4,7,10,15,18,21,24,28,31,34-undecazatricyclo[34.3.0.012,15]nonatriacontane-33,1'-cyclopentane]-2,5,8,11,16,19,22,25,29,32,35-undecaone
CP02: (3S,9S,12S,17S,20S,23S,27S,30S,36S)-30-cyclopentyl-3-[2-[3,5-difluoro-4-(trifluoromethyl)phenyl]ethyl]-10-ethyl-23-isobutyl-N,N,7,17,18,24,28,31-octamethyl-20-[(1S)-1-methylpropy1]-2,5,8,11,16,19,22,25,29,32,35-undecaoxo-9-(p-trylmethyl)spiro[1,4,7,10,15,18,21,24,28,31,34-undecazatricyclo[34.3.0.012,15]nonatriacontane-33,1'-cyclopentane]-27-carboxamide
CP03: (2S,8S,12R,14S,20S,23S,27S,30S,36S,38Z)-20-cyclopentyl-8-[2-[3,5-difluoro-4-(trifluoromethyl)phenyl]ethyl]-12-ethoxy-27-isobutyl-N,N,4,19,22,26,32,35-octamethyl-30-[(1S)-1-methylpropyl]-3,6,9,15,18,21,25,28,31,34,42-undecaoxo-2-[[4-(trifluoromethyl)phenyl]methyl]spiro[1,4,7,10,16,19,22,26,29,32,35-undecazatricyclo[34.5.1.010,14]dotetracont-38-ene-17,1'-cyclopentane]-23-carboxamide CP04: (1S,4S,10S,13S,17S,20S,26S,28R,32S,38S,42Z)-20-cyclopentyl-28-ethoxy-32-[2-[3-methoxy-4-(tritluoromethyl)phenyl]ethyl]-N,N,2,14,18,21,24,36-octamethyl-10-[(1S)-1-methylpropyl]-3,9,12,15,19,22,25,31,34,37,45-undecaoxo-13-propyl-38-[[4-(trifluoromethyl)phenyl]methyl]spiro[2,8,11,14,18,21,24,30,33,36,39-undecazatetracyclo[37.5.1.04,8.026,30]pentatetracont-42-ene-23,1'-cyclobutane]-17-carboxamide

### [ClogP]

CP01: 14.899
CP02: 14.504
CP03: 15.204
CP04: 15.127

### [Molecular weight]

CP01: 1478.2
CP02: 1437.7
CP03: 1547.7
CP04: 1553.8

### [Production Example 2] Production of solid dispersion

### (Production Example 2-1) Production of solid dispersion using HPMCAS

A peptide and HPMCAS were added to acetone or tetrahydrofuran such that the weight ratio of the peptide and HPMCAS was 1 : 2 and the solid concentration was 12 wt/vol%, thereby preparing a uniform suspension. The suspension was spray-dried to obtain a solid dispersion.

### (Production Example 2-2) Production of solid dispersion using Eudragit L100

A peptide and Eudragit L100 were added to ethanol such that the weight ratio of the peptide and Eudragit L100 was 1 : 2 and the solid concentration was 8 wt/vol%, thereby preparing a uniform solution. The suspension was spray-dried to obtain a solid dispersion.

### [Evaluation Example] Evaluation of solubility and solid state

### (Evaluation 1-1)

X-ray powder diffraction measurement of samples obtained in Reference Examples, Examples and Comparative Examples were performed under the following conditions.
Measurement apparatus: D8 Discover, 2D VÅNTEC-500 solid state detector (manufactured by Bruker)
Radiation source: CuKα
Tube voltage and tube current: 40 kV and 40 mA or 50 kV and 1,000 µA
Measurement range: 5 to 31
Exposure time: 40 to 600 seconds

### (Evaluation Example 1-2) Implementation of solubility test

A solubility test on the sample obtained in each of Examples, Comparative Examples and Reference Examples was conducted at 37°C, and the solubility under the conditions was calculated by an internal standard method using an ultra-high-performance liquid chromatography method with a photodiode array detector.

### [Examples, Comparative Examples and Reference Examples] Preparation and evaluation of pharmaceutical composition

### (Example 1, Comparative Examples 1-1 and 1-2 and Reference Example 1)

DMSO solutions (50 µL) whose compositions are shown in Table 2 were each freeze-dried to prepare powder in which the cyclic peptide CP02 was made amorphous. To each of the obtained samples, FaSSIF (50 µL) was added, and the mixture was shaken for 4 hours, and then filtered, followed by measurement of the solubility. Table 3 shows the results. As an index of the solubilizing effect, a solubility difference from Reference Example 1 was calculated. Table 3 shows the results. The sum of the calculated solubility differences in Comparative Examples and the calculated solubility difference in Example were compared to estimate the amount of improvement in solubilizing effect.

The results of Comparative Examples 1-1 and 1-2 show that HPMCAS and SLS alone exhibited solubilizing effects of 7.2 µg/mL and 231.4 µg/mL, respectively. On the other hand, the results of Example 1 show that a combination of SLS and HPMCAS exhibited a solubilizing effect higher by 82.6 µg/mL than the summed value of effects of SLS and HPMCAS added alone, and produced an about 1.3-fold synergistic effect.

**[Table 2]**

| | Reference Example 1 | Comparative Example 1-1 | Comparative Example 1-2 | Example 1 |
|---|---|---|---|---|
| CP02 (mg/mL) | 0.25 | 0.25 | 0.50 | 0.50 |
| SLS (mg/mL) | | | 0.50 | 0.50 |
| HPMCAS (mg/mL) | | 0.5 | | 1.0 |

**Table 3]**

| | Reference Example 1 | Comparative Example 1-1 | Comparative Example 1-2 | Example 1 |
|---|---|---|---|---|
| Solubility (µg/mL) | 16.3 | 23.5 | 247.7 | 337.5 |
| Difference from Reference Example 1 (µg/mL) | - | 7.2 | 231.4 | 321.2 |

### (Example 2, Comparative Examples 2-1 and 2-2 and Reference Example 2)

DMSO solutions (50 µL) whose compositions are shown in Table 4 were each freeze-dried to prepare powder in which the cyclic peptide CP03 was made amorphous. To each of the obtained samples, FaSSIF (50 µL) was added, and the mixture was shaken for 4 hours, and then filtered, followed by measurement of the solubility. Table 5 shows the results. As an index of the solubilizing effect, a solubility difference from Reference Example 2 was calculated. Table 5 shows the results. The sum of the calculated solubility differences in Comparative Examples and the calculated solubility difference in Example were compared to estimate the amount of improvement in solubilizing effect. The results of Comparative Examples 2-1 and 2-2 show that HPMCAS and SLS alone exhibited solubilizing effects of 4.3 µg/mL and 171.6 µg/mL, respectively. On the other hand, the results of Example 2 show that a combination of SLS and HPMCAS exhibited a solubilizing effect higher by 156.0 µg/mL than the summed value of effects of SLS and HPMCAS added alone, and produced an about 1.9-fold synergistic effect.

**[Table 4]**

| | Reference Example 2 | Comparative Example 2-1 | Comparative Example 2-2 | Example 2 |
|---|---|---|---|---|
| CP03 (mg/mL) | 0.25 | 0.25 | 0.50 | 0.50 |
| SLS (mg/mL) | | | 0.50 | 0.50 |
| HPMCAS (mg/mL) | | 0.50 | | 1.0 |

**[Table 5]**

| | Reference Example 2 | Comparative Example 2-1 | Comparative Example 2-2 | Example 2 |
|---|---|---|---|---|
| Solubility (µg/mL) | 8.0 | 12.3 | 179.6 | 339.9 |
| Difference from Reference Example 2 (µg/mL) | - | 4.3 | 171.6 | 331.9 |

### (Example 3, Comparative Examples 3-1 and 3-2 and Reference Example 3)

DMSO solutions (50 µL) whose compositions are shown in Table 6 were each freeze-dried to prepare powder in which the cyclic peptide CP04 was made amorphous. To each of the obtained samples, FaSSIF (50 µL) was added, and the mixture was shaken for 4 hours, and then filtered, followed by measurement of the solubility. Table 7 shows the results. As an index of the solubilizing effect, a solubility difference from Reference Example 3 was calculated. Table 7 shows the results. The sum of the calculated solubility differences in Comparative Examples and the calculated solubility difference in Example were compared to estimate the amount of improvement in solubilizing effect. The results of Comparative Examples 3-1 and 3-2 show that HPMCAS and SLS alone exhibited solubilizing effects of 3.9 µg/mL and 72.2 µg/mL, respectively. On the other hand, the results of Example 3 show that a combination of SLS and HPMCAS exhibited a solubilizing effect higher by 54.7 µg/mL than the summed value of effects of SLS and HPMCAS added alone, and produced an about 1.7-fold synergistic effect.

**Table 6]**

| | Reference Example 3 | Comparative Example 3-1 | Comparative Example 3-2 | Example 3 |
|---|---|---|---|---|
| CP04 (mg/mL) | 0.25 | 0.25 | 0.25 | 0.25 |
| SLS (mg/mL) | | | 0.50 | 0.50 |
| HPMCAS (mg/mL) | | 0.50 | | 0.50 |

**[Table 7]**

| | Reference Example 3 | Comparative Example 3-1 | Comparative Example 3-2 | Example 3 |
|---|---|---|---|---|
| Solubility (µg/mL) | 0.5 | 4.4 | 72.7 | 131.3 |
| Difference from Reference Example 3 (µg/mL) | | 3.9 | 72.2 | 130.8 |

### (Comparative Example 4 and Examples 4-1 to 4-4) Measurement of solubility of CP02 crystal

HPMCAS and SLS were dissolved in FaSSIF at concentrations shown in Table 8. To the C-type crystal of CP02, each of the solutions was added, and the mixture was shaken overnight. Coarse particles were precipitated by centrifugation, and the supernatant was then filtered through a 0.45 µm filter, followed by measurement of the solubility. Table 9 shows the results. In Examples 4-1 to 4-4, a solubilizing effect higher by 1.30 to 1.85 µg/mL than that in Comparative Example 4 was exhibited, and an about 1.7- to 2.1-fold solubility improvement was confirmed. It was found from the present results that a combination of HPMCAS and SLS improved the solubilizing effect even in a cyclic peptide in a crystalline state. Further, the solid residue of Example 4-4 was subjected to powder X-ray diffraction measurement, and the results showed that the C-type crystal was maintained. Therefore, it was confirmed that the solubility improvement was not due to the crystallization suppressing action of HPMCAS, but due to the combination of HPMCAS and SLS.

**Table 8]**

| | Comparative Example 4 | Example 4-1 | Example 4-2 | Example 4-3 | Example 4-4 |
|---|---|---|---|---|---|
| SLS (mg/mL) | 0.3 | 0.3 | 0.3 | 0.3 | 0.3 |
| HPMCAS (mg/mL) | 0 | 0.1 | 0.5 | 1.0 | 2.0 |

**[Table 9]**

| | Comparative Example 4 | Example 4-1 | Example 4-2 | Example 4-3 | Example 4-4 |
|---|---|---|---|---|---|
| Solubility (µg/mL) | 1.76 | 3.14 | 3.14 | 3.06 | 3.61 |
| Solubility ratio with respect to Comparative Example 4 | - | 1.8 | 1.8 | 1.7 | 2.1 |
| Solubility difference from Comparative Example 4 (µg/mL) | | 1.38 | 1.38 | 1.30 | 1.85 |

### (Reference Example 5, Examples 5-1 to 5-3 and Comparative Examples 5-1 to 5-4)

### Measurement of solubility of CP03

SLS was dissolved in FaSSIF at 0.47 mg/mL to prepare a FaSSIF-SLS solution. LC was dissolved in FaSSIF at 1.57 mg/mL to prepare a FaSSIF-LC solution. To CP03 (1 mg) in an amorphous form or a solid dispersion (3 mg) produced under the conditions of Production Example 2-1 or 2-2, FaSSIF, a FaSSIF-SLS solution or a FaSSIF-LC solution (1 mL) was added, and the mixture was shaken for 2 hours. Coarse particles were precipitated by centrifugation, and the supernatant was then filtered through a 0.45 µm filter, followed by measurement of the solubility. As an index of the solubilizing effect, a solubility difference from Reference Example 5 was calculated. Table 10 shows the results. The sum of the calculated solubility differences in Comparative Examples and the calculated solubility difference in Example were compared to estimate the amount of improvement in solubilizing effect. The results of Comparative Examples 5-1 and 5-3 show that HPMCAS and SLS alone exhibited solubilizing effects of 19.5 µg/mL and 157.1 µg/mL, respectively. On the other hand, the results of Example 5-1 show that a combination of SLS and HPMCAS exhibited a solubilizing effect higher by 117.0 µg/mL than the summed value of effects of SLS and HPMCAS added alone, and produced an about 1.7-fold synergistic effect.

The results of Comparative Example 5-2 show that LC alone exhibited a solubilizing effect of 70.6 µg/mL. On the other hand, the results of Example 5-2 show that a combination of LC and HPMCAS exhibited a solubilizing effect higher by 113.7 µg/mL than the summed value of effects of LC and HPMCAS added alone, and produced an about 2.3-fold synergistic effect.

The results of Comparative Example 5-4 show that Eudragit L100 exhibited a solubilizing effect of 48.5 µg/mL. On the other hand, the results of Example 5-3 show that a combination of SLS and Eudragit L100 exhibited a solubilizing effect higher by 112.9 µg/mL than the summed value of effects of SLS and Eudragit L100 added alone, and produced an about 1.5-fold synergistic effect.

Further, the solid residue in the solubility measurement was subjected to powder X-ray diffraction measurement, and the results showed that a diffraction peak was not observed under any conditions, and the residue was amorphous. It was confirmed from the present results that the solubility improvement by the addition of HPMCAS or Eudragit L100 was not due to the crystallization suppressing action, but due to enhancement of the solubilizing effect by a combination of any one polymer selected from the group consisting of HPMCAS and Eudragit L100 and one surfactant selected from the group consisting of SLS and LC.

**[Table 10]**

| | Reference Example 5 | Comparative Example 5-1 | Comparative Example 5-2 | Comparative Example 5-3 | Comparative Example 5-4 | Example 5-1 | Example 5-2 | Example 5-3 |
|---|---|---|---|---|---|---|---|---|
| Test substance | Amorphous with only CP03 | Amorphous with only CP03 | Amorphous with only CP03 | Amorphous with CP03 and HPMCAS (solid dispersion) | Amorphous with CP03 and Eudragit L100 (solid dispersion) | Amorphous with CP03 and HPMCAS (solid dispersion) | Amorphous with CP03 and HPMCAS (solid dispersion) | Amorphous with CP03 and Eudragit L100 (solid dispersion) |
| Dissolving solvent | FaSSIF | FaSSIF-SLS solution | FaSSIF-LC solution | FaSSIF | FaSSIF | FaSSIF-SLS solution | FaSSIF-LC solution | FaSSIF-SLS solution |
| Solubility (µg/mL) | 12.3 | 169.4 | 82.9 | 31.8 | 60.8 | 305.9 | 116.1 | 330.8 |
| Difference from Reference Example 5 (µg/mL) | - | 157.1 | 70.6 | 19.5 | 48.5 | 293.6 | 203.8 | 318.5 |

### (Comparative Example 6 and Examples 6-1 to 6-2) Measurement of solubility of CP04

SLS was dissolved in FaSSIF at 0.47 mg/mL to prepare a FaSSIF-SLS solution. To CP04 (1 mg) in an amorphous form or a solid dispersion (3 mg) produced under the conditions of Production Example 2-1 or 2-2, a FaSSIF-SLS solution (1 mL) was added, and the mixture was shaken for 2 hours. Coarse particles were precipitated by centrifugation, and the supernatant was then filtered through a 0.45 µm filter, followed by measurement of the solubility. Table 11 shows the results. SLS alone exhibited a solubilizing effect of 75.0 µg/mL. On the other hand, a combination of SLS and HPMCAS exhibited a solubilizing effect of 114.0 µg/mL, and produced an about 1.5-fold synergistic effect. A combination of SLS and Eudragit L100 exhibited a solubilizing effect of 124.2 µg/mL, and produced an about 1.7-fold synergistic effect.

Further, the solid residue in the solubility measurement was subjected to powder X-ray diffraction measurement, and the results showed that a diffraction peak was not observed under any conditions, and the residue was amorphous. It was confirmed from the present results that the solubility improvement by the addition of HPMCAS or Eudragit L100 was not due to the crystallization suppressing action, but due to enhancement of the solubilizing effect by HPMCAS or Eudragit L100.

**[Table 11]**

| | Comparative Example 6 | Example 6-1 | Example 6-2 |
|---|---|---|---|
| Test substance | Amorphous with CP04 | Amorphous with CP04 and HPMCAS (solid dispersion) | Amorphous with CP04 and Eudragit L100 (solid dispersion) |
| Dissolving solvent | FaSSIF-SLS solution | FaSSIF-SLS solution | FaSSIF-SLS solution |
| Solubility (µg/mL) | 75.0 | 114.0 | 124.2 |

### (Example 7, Comparative Examples 7-1 and 7-2 and Reference Example 7)

Fifty µL of each of DMSO solutions whose compositions are shown in Table 12 were freeze-dried to prepare powder in which the cyclic peptide CP01 was made amorphous. To each of the obtained samples, 50 µL of FaSSIF was added, and the mixture was shaken for 4 hours, and then filtered, followed by measurement of the solubility. Table 13 shows the results. As an index of the solubilizing effect, a solubility difference from Reference Example 7 was calculated. Table 13 shows the results. The sum of the calculated solubility differences in Comparative Examples and the calculated solubility difference in Example were compared to estimate the amount of improvement in solubilizing effect. The results of Comparative Examples 7-1 and 7-2 show that HPMCAS and SLS alone exhibited solubilizing effects of 6.1 µg/mL and 219.1 µg/mL, respectively. On the other hand, the results of Example 7 show that a combination of SLS and HPMCAS exhibited a solubilizing effect higher by 114.3 µg/mL than the summed value of effects of SLS and HPMCAS added alone, and produced an about 1.5-fold synergistic effect.

**[Table 12]**

| | Reference Example 7 | Comparative Example 7-1 | Comparative Example 7-2 | Example 7 |
|---|---|---|---|---|
| CP01 (mg/mL) | 0.25 | 0.25 | 0.5 | 0.5 |
| SLS (mg/mL) | | | 0.5 | 0.5 |
| HPMCAS (mg/mL) | | 0.5 | | 1.0 |

**[Table 13]**

| | Reference Example 7 | Comparative Example 7-1 | Comparative Example 7-2 | Example 7 |
|---|---|---|---|---|
| Solubility (µg/mL) | 15.7 | 21.8 | 234.8 | 355.2 |
| Difference from Reference Example 7 (µg/mL) | - | 6.1 | 219.1 | 339.5 |

### (Reference Example 8, Examples 8-1 and 8-2 and Comparative Examples 8-1 to 8-3)

### Measurement of solubility of CP01

SLS was dissolved in FaSSIF at 0.3 mg/mL to prepare a FaSSIF-SLS solution. LC was dissolved in FaSSIF at 0.3 mg/mL to prepare a FaSSIF-LC solution. To CP01 (1 mg) in an amorphous form or a solid dispersion (4.5 mg) produced under the conditions of Production Example 2-1, 1 mL of FaSSIF, a FaSSIF-SLS solution or a FaSSIF-LC solution was added, and the mixture was shaken for 2 hours. Coarse particles were precipitated by centrifugation, and the supernatant was then filtered through a 0.45 µm filter, followed by measurement of the solubility. As an index of the solubilizing effect, a solubility difference from Reference Example 8 was calculated. Table 14 shows the results. The sum of the calculated solubility differences in Comparative Examples and the calculated solubility difference in Example were compared to estimate the amount of improvement in solubilizing effect. The results of Comparative Examples 8-1 and 8-3 show that SLS and HPMCAS alone exhibited solubilizing effects of 162.2 µg/mL and 40.0 µg/mL, respectively. On the other hand, the results of Example 8-1 show that a combination of SLS and HPMCAS exhibited a solubilizing effect higher by 164.3 µg/mL than the summed value of effects of SLS and HPMCAS added alone, and produced an about 1.8-fold synergistic effect.

The results of Comparative Example 8-2 show that LC alone exhibited a solubilizing effect of 41.5 µg/mL. On the other hand, the results of Example 8-2 show that a combination of LC and HPMCAS exhibited a solubilizing effect higher by 45.4 µg/mL than the summed value of effects of LC and HPMCAS added alone, and produced an about 1.6-fold synergistic effect.

Further, the solid residue in the solubility measurement was subjected to powder X-ray diffraction measurement, and the results showed that a diffraction peak was not observed under any conditions, and the residue was amorphous. It was confirmed from the present results that the solubility improvement by the addition of HPMCAS was not due to the crystallization suppressing action, but due to enhancement of the solubilizing effect by a combination of HPMCAS and SLS or LC.

**[Table 14]**

| | Reference Example 8 | Comparative Example 8-1 | Comparative Example 8-2 | Comparative Example 8-3 | Example 8-1 | Example 8-2 |
|---|---|---|---|---|---|---|
| Test substance | Amorphous with only CP01 | Amorphous with only CP01 | Amorphous with only CP01 | Amorphous with CP01 and HPMCAS (solid dispersion) | Amorphous with CP01 and HPMCAS (solid dispersion) | Amorphous with CP01 and HPMCAS (solid dispersion) |
| Dissolving solvent | FaSSIF | FaSSIF-SLS solution | FaSSIF-LC solution | FaSSIF | FaSSIF-SLS solution | FaSSIF-LC solution |
| Solubility (µg/mL) | 26.1 | 188.3 | 67.6 | 66.1 | 392.6 | 153.0 |
| Difference from Example 8 (µg/mL) | - | 162.2 | 41.5 | 40.0 | 366.5 | 126.9 |

### (Examples 9-1 to 9-3, Comparative Examples 9-2 to 9-4 and Reference Example 9)

DMSO solutions (50 µL) whose compositions are shown in Table 15 were each freeze-dried to prepare powder in which the cyclic peptide CP01 was made amorphous. To each of the obtained samples, FaSSIF (50 µL) was added, and the mixture was shaken for 4 hours, and then filtered, followed by measurement of the solubility. Table 16 shows the results. As an index of the solubilizing effect, a solubility difference from Reference Example 9 was calculated. Table 16 shows the results. The sum of the calculated solubility differences in Comparative Examples and the calculated solubility difference in Example were compared to estimate the amount of improvement in solubilizing effect.

The results of Comparative Example 9-1 show that SLS alone exhibited a solubilizing effect of 200.8 µg/mL. The results of Comparative Example 9-5 show that Soluplus alone exhibited a solubilizing effect of 6.1 µg/mL. Further, the results of Example 9-1 show that a combination of SLS and Soluplus exhibited a solubilizing effect higher by 33.1 µg/mL than the summed value of effects of SLS and Soluplus added alone, and produced an about 1.2-fold synergistic effect.

On the other hand, the results of Comparative Examples 9-2 to 9-4 show that PVP K30, Kollidone VA64 and Eudragit L100 did not exhibit a solubilizing effect when a solid dispersion obtained by freeze-drying a DMSO solution of a peptide and a polymer was used. The results of Comparative Examples 9-6 to 9-8 show that when a solid dispersion obtained by freeze-drying a DMSO solution of a peptide and a polymer was used, even a combination of SLS and PVP K30, Kollidone VA64 or Eudragit L100 rather made the solubility lower than the summed value of effects of SLS and PVP K30, Kollidone VA64 or Eudragit L100 alone, and did not produce a synergistic solubilizing effect.

**[Table 15]**

| | Reference Example 9 | Comparative Example 9-1 | Comparative Example 9-2 | Comparative Example 9-3 | Comparative Example 9-4 | Comparative Example 9-5 | Comparative Example 9-6 | Comparative Example 9-7 | Comparative Example 9-8 | Example 9-1 |
|---|---|---|---|---|---|---|---|---|---|---|
| CP01 (mg/mL) | 0.5 | 0.5 | 0.5 | 0.5 | 0.5 | 0.5 | 0.5 | 0.5 | 0.5 | 0.5 |
| SLS (mg/mL) | | 0.3 | | | | | 0.3 | 0.3 | 0.3 | 0.3 |
| PVP K30 (mg/mL) | | | 0.5 | | | | 0.5 | | | |
| Kollidone VA64 (mg/mL) | | | | 0.5 | | | | 0.5 | | |
| Eudragit L100 (mg/mL) | | | | | 0.5 | | | | 0.5 | |
| Soluplus (mg/mL) | | | | | | 0.5 | | | | 0.5 |

**[Table 16]**

| | Reference Example 9 | Comparative Example 9-1 | Comparative Example 9-2 | Comparative Example 9-3 | Comparative Example 9-4 | Comparative Example 9-5 | Example 9-1 | Example 9-2 | Example 9-3 | Example 9-4 |
|---|---|---|---|---|---|---|---|---|---|---|
| Solubility (µg/mL) | 24.5 | 225.3 | 20.9 | 19.8 | 24.7 | 30.6 | 209.5 | 197.1 | 223.8 | 264.5 |
| Difference from Reference Example 9 (µg/mL) | - | 200.8 | -3.6 | -4.7 | 0.2 | 6.1 | 185.0 | 172.6 | 199.3 | 240.0 |

### (Examples 10-1 to 10-3 and Comparative Examples 10-2 to 10-4)

DMSO solutions (50 µL) whose compositions are shown in Table 17 were each freeze-dried to prepare powder in which the cyclic peptide CP01 was made amorphous. To each of the obtained samples, FaSSIF (50 µL) was added, and the mixture was shaken for 4 hours, and then filtered, followed by measurement of the solubility. Table 18 shows the results. As an index of the solubilizing effect, a solubility difference from Reference Example 9 was calculated. Table 18 shows the results. The sum of the calculated solubility differences in Comparative Examples and the calculated solubility difference in Example were compared to estimate the amount of improvement in solubilizing effect.

The results of Comparative Examples 10-1 to 10-4 show that HPMCAS, DKester, HCO 60 and Vitamin E TPGS alone exhibited solubilizing effects of 0.8 µg/mL, 37.3 µg/mL, 9.0 µg/mL and 69.5 µg/mL, respectively. The results of Example 10-1 show that a combination of DKester and HPMCAS exhibited a solubilizing effect higher by 34.6 µg/mL than the summed value of effects of DKester and HPMCAS added alone, and produced an about 1.9-fold synergistic effect. The results of Example 10-2 show that a combination of HCO 60 and HPMCAS exhibited a solubilizing effect higher by 14.4 µg/mL than the summed value of effects of HCO 60 and HPMCAS added alone, and produced an about 2.5-fold synergistic effect. The results of Example 10-3 show that a combination of Vitamin E TPGS and HPMCAS exhibited a solubilizing effect higher by 11.8 µg/mL than the summed value of effects of Vitamin E TPGS and HPMCAS added alone, and produced an about 1.2-fold synergistic effect.

**Table 17]**

| | Reference Example 9 | Comparative Example 10-1 | Comparative Example 10-2 | Comparative Example 10-3 | Comparative Example 10-4 | Example 10-1 | Example 10-2 | Example 10-3 |
|---|---|---|---|---|---|---|---|---|
| CP01 (mg/ml) | 0.5 | 0.5 | 0.5 | 0.5 | 0.5 | 0.5 | 0.5 | 0.5 |
| DK ester (mg/mL) | | | 0.3 | | | 0.3 | | |
| HCO60 (mg/mL) | | | | 0.3 | | | 0.3 | |
| Vitamin E TPGS (mg/mL) | | | | | 0.3 | | | 0.3 |
| HPMCAS (mg/mL) | | 0.5 | | | | 0.5 | 0.5 | 0.5 |

**[Table 18]**

| | Reference Example 9 | Comparative Example 10-1 | Comparative Example 10-2 | Comparative Example 10-3 | Comparative Example 10-4 | Example 10-1 | Example 10-2 | Example 10-3 |
|---|---|---|---|---|---|---|---|---|
| Solubility (µg/mL) | 24.5 | 25.3 | 61.8 | 33.5 | 94.0 | 97.2 | 48.7 | 106.6 |
| Difference from Reference Example 9 (µg/mL) | - | 0.8 | 37.3 | 9.0 | 69.5 | 71.9 | 23.4 | 81.3 |

## Claims

1. A composition comprising: a solid dispersion that is a spray-dried product of a mixture containing a peptide and one or more polymers selected from the group consisting of the following (IV) to (VIII); and one or more surfactants selected from the group consisting of the following (I) to (III):
(I) an anionic surfactant;
(II) an amphoteric surfactant;
(III) a nonionic surfactant;
(IV) hydroxypropyl methyl cellulose or a derivative thereof;
(V) a copolymer of a (meth)acrylic acid alkyl ester and at least one monomer selected from the group consisting of a (meth)acrylic acid, a (meth)acrylic acid alkyl ester, a (meth)acrylic acid alkylaminoalkyl ester and a (meth)acrylic acid ammonioalkyl ester;
(VI) copovidone;
(VII) a polyvinylcaprolactam-polyvinyl acetate-polyethylene glycol graft copolymer; and
(VIII) polyvinylpyrrolidone.

2. A composition comprising: a solid dispersion containing a peptide and one or more polymers selected from the group consisting of the following (IV) and (VII); and one or more surfactants selected from the group consisting of the following (I) to (III):
(I) an anionic surfactant;
(II) an amphoteric surfactant;
(III) a nonionic surfactant;
(IV) hydroxypropyl methyl cellulose or a derivative thereof; and
(VII) a polyvinylcaprolactam-polyvinyl acetate-polyethylene glycol graft copolymer.

3. A composition comprising: a peptide (CP02) represented by the following formula, one or more surfactants selected from the group consisting of the following (I) to (III); and one or more polymers selected from the group consisting of the following (IV), (V) and (VII): 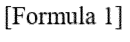 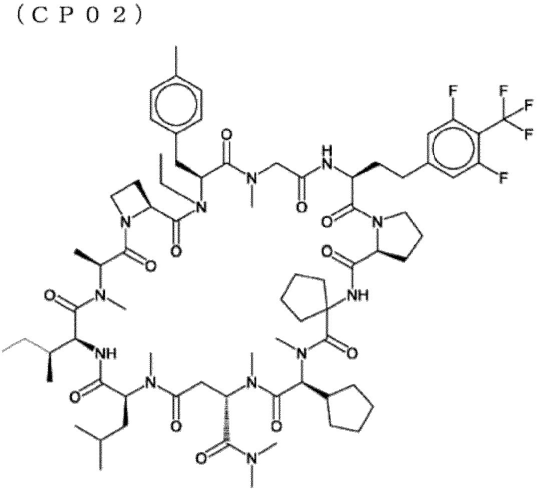
(I) an anionic surfactant;
(II) an amphoteric surfactant;
(III) a nonionic surfactant;
(IV) hydroxypropyl methyl cellulose or a derivative thereof; and
(V) a copolymer of a (meth)acrylic acid alkyl ester and at least one monomer selected from the group consisting of a (meth)acrylic acid, a (meth)acrylic acid alkyl ester, a (meth)acrylic acid alkylaminoalkyl ester and a (meth)acrylic acid ammonioalkyl ester; and
(VII) a polyvinylcaprolactam-polyvinyl acetate-polyethylene glycol graft copolymer.

4. The composition according to claim 1 or 2, wherein the number of amino acid residues forming the peptide is 5 or more and 30 or less.

5. The composition according to claim 1 or 2, wherein the number of N-substituted amino acid residues contained in the peptide is 1 or more.

6. The composition according to any one of claims 1 to 5, wherein the surfactant is sodium lauryl sulfate.

7. The composition according to any one of claims 1 to 5, wherein the surfactant is lauroyl-L-carnitine.

8. The composition according to any one of claims 1 to 7, wherein the polymer is hydroxypropyl methyl cellulose or a derivative thereof.

9. The composition according to any one of claims 1 to 7, wherein the polymer is hydroxypropyl methyl cellulose acetate succinate (HPMCAS).

10. The composition according to any one of claims 1 to 7, wherein the polymer is a copolymer of a (meth)acrylic acid alkyl ester and at least one monomer selected from the group consisting of (meth)acrylic acid, a (meth)acrylic acid alkyl ester, a (meth)acrylic acid alkylaminoalkyl ester and a (meth)acrylic acid ammonioalkyl ester.

11. The composition according to any one of claims 1 to 5, wherein the polymer is a copolymer of methyl methacrylate and methacrylic acid.

12. The composition according to any one of claims 1 to 5, wherein the surfactant is an anionic surfactant, and the polymer is hydroxypropyl methyl cellulose or a derivative thereof.

13. The composition according to any one of claims 1 to 5, wherein the surfactant is an anionic surfactant, and the polymer is a copolymer of a (meth)acrylic acid alkyl ester and at least one monomer selected from the group consisting of (meth)acrylic acid, a (meth)acrylic acid alkyl ester, a (meth)acrylic acid alkylaminoalkyl ester and a (meth)acrylic acid ammonioalkyl ester.

14. The composition according to any one of claims 1 to 5, wherein the surfactant is an amphoteric surfactant, and the polymer is hydroxypropyl methyl cellulose or a derivative thereof.

15. The composition according to any one of claims 1 to 5, wherein the surfactant is an amphoteric surfactant, and the polymer is a copolymer of a (meth)acrylic acid alkyl ester and at least one monomer selected from the group consisting of (meth)acrylic acid, a (meth)acrylic acid alkyl ester, a (meth)acrylic acid alkylaminoalkyl ester and a (meth)acrylic acid ammonioalkyl ester.
